(19) Europäisches Patentamt
European Patent Office
Office européen des brevets

(11) **EP 4 635 518 A1**

(12) **EUROPEAN PATENT APPLICATION**

(43) Date of publication:
**22.10.2025 Bulletin 2025/43**

(21) Application number: **25158185.6**

(22) Date of filing: **17.02.2025**

(51) International Patent Classification (IPC):
*A61K 47/69* (2017.01)    *A61K 9/00* (2006.01)
*A61K 47/68* (2017.01)    *A61P 27/02* (2006.01)

(52) Cooperative Patent Classification (CPC):
**A61K 47/6929; A61K 9/0019; A61K 9/0048;
A61K 9/5123; A61K 47/643; A61K 47/6843;
A61K 47/6887; A61K 47/6923; A61P 27/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.04.2024 EP 24171280**

(71) Applicant: **Universität Münster
48149 Münster (DE)**

(72) Inventors:
• **Langer, Klaus
48149 Münster (DE)**
• **Nitschke, Yvonne
48149 Münster (DE)**
• **Rutsch, Frank
48149 Münster (DE)**

(74) Representative: **Schiweck Weinzierl Koch
Patentanwälte Partnerschaft mbB
Ganghoferstraße 68 B
80339 München (DE)**

(54) **NANOPARTICLE FOR USE IN A PROPHYLAXIS OR IN A TREATMENT OF AGE-RELATED MACULAR DEGENERATION AND/OR OPTIC NERVE HEAD DRUSEN**

(57) The present invention provides a nanoparticle comprising a scaffold comprising a biodegradable material, an antibody, which is able to bind to a component of a Bruch's membrane, a component of a subretinal pigment epithelial deposit, or a component of an optic nerve head, and an anti-calcifying agent for use in a prophylaxis or in a treatment of age-related macular degeneration and/or optic nerve head drusen of an eye. Said prophylaxis or treatment is accomplished by the prophylaxis or the treatment of a calcified subretinal pigment epithelium deposit and/or calcified drusen of an eye. Additionally, the present invention provides a pharmaceutical composition comprising said nanoparticle and one or more pharmaceutical acceptable excipient(s). Said pharmaceutical composition is for use in a prophylaxis or in a treatment of age-related macular degeneration and/or optic nerve head drusen.

EP 4 635 518 A1

**Description**

**CROSS REFERENCE TO RELATED APPLICATION**

The present application claims the benefit of priority of EP Patent Application No. 24171280.1 filed 19 April 2024, the content of which is hereby incorporated by reference in its entirety for all purposes.

**TECHNICAL FIELD OF THE INVENTION**

The present invention provides a nanoparticle comprising a scaffold comprising a biodegradable material, an antibody, which is able to bind to a component of a Bruch's membrane, a component of a subretinal pigment epithelial deposit, or a component of optic nerve head, and an anti-calcifying agent for use in a prophylaxis or in a treatment of age-related macular degeneration and/or optic nerve head drusen of an eye. The prophylaxis or the treatment of age-related macular degeneration and/or optic nerve head drusen is accomplished by the prophylaxis or the treatment of calcified subretinal pigment epithelium deposit and/or calcified drusen of the eye. Additionally, the present invention provides a pharmaceutical composition comprising said nanoparticle and one or more pharmaceutical acceptable excipient(s). Said pharmaceutical composition is for use in a prophylaxis or in a treatment of age-related macular degeneration and/or optic nerve head drusen of the eye.

**BACKGROUND**

A Bruch's membrane (BM) is a pentalaminar structure located between a retinal pigment epithelium (RPE) and fenestrated choroidal capillaries of the eye. The Bruch's membrane is an elastin- and collagen- rich matrix that acts as a molecular sieve: it partly regulates an exchange of biomolecules, nutrients, oxygen, fluids and metabolic waste products between a retina and a systemic circulation (Booij et al., 2010). The Bruch's membrane consists of five layers, namely, retinal pigment epithelium basal lamina, (RPE-BL), inner collagenous layer (ICL), elastic layer (EL), outer collagenous layer (ICL), and choriocapillaris basal lamina (Chc-BL). The elastic layer predominantly contains elastin in addition to fibronectin and other proteins and collagen VI (Booij et al., 2010). The functions of Bruch's membrane include 1) the regulation of the diffusion of (bio-) molecules between the choroid and RPE, 2) providing physical support for RPE cell adhesion, migration and perhaps differentiation, 3) acting as a division barrier, restricting choroidal and retinal cell migration (Booij et al., 2010).

The Bruch's membrane and a subretinal pigment epithelial space could be calcified as a symptom of various eye diseases, including age-related macular degeneration (AMD). In optic disc drusen (= optic nerve head drusen), calcifications occur at the lamina cribrosa, an elastin rich area at the optic nerve head (Palmer et al., 2018).

In 1992, van der Schaft et al. studied 182 human maculae older than 33 years of age, and found calcium deposition in BM in 59% of the samples. The presence of BM calcification was positively correlated with age, but not with age related macular degeneration (AMD) in this study (van der Schaft et al., 1992). However, a study by Spraul et al. in 1999 demonstrated a significant correlation between BM calcification and AMD. In this study, the authors concluded that "calcification of BM is an important factor that increases the brittleness of BM and enhances the chances of fibrovascular ingrowth" (Spraul et al., 1999). In a study by Biesemeier et al., five of six AMD donors, but only one of the seven controls showed nanocrystalline hydroxyapatite (HAP) calcifications at the level of the BM. The authors demonstrated that these calcifications were hydroxyapatite based, located in the elastic layer of BM and showed a "cage-like" structure, which "would facilitate additional storage of iron and lead in the calcified areas". The authors proposed that the calcification acts as a barrier, decreasing the permeability of BM. They concluded that, together with the accumulating heavy metal iron and lead "this can facilitate degeneration of the overlying RPE and retina" (Biesemeier et al., 2015).

Independently, Thompson et al. identified hydroxyapatite spherules with cholesterol-containing cores in sub-retinal pigment epithelial (sub-RPE) deposits with proteins, including vitronectin, beta-amyloid and complement factor H bound to these structures in human eyes. They observed HAP spherule formation on the surface of lipid droplets in the inner aspect of Bruch's membrane and concluded that HAP can be directly formed in physiological concentrations of calcium and phosphate at physiological pH and that this process may occur in the sub-RPE space (Thompson et al., 2015; Boskey et al., 1976).

Apart from the "spherules", which range from 0.5 to 10 $\mu$m in diameter, with the most frequent size of the distribution around 1.5 $\mu$m, a recent study by Tan et al. identified a subset of HAP deposits, which are larger (tens of micrometers in size) than the spherules and termed "calcified nodules" in retinal drusen. The presence of calcified drusen was associated with increased risk (odds ratio 6,4) of progression to advanced AMD within one year, providing good evidence for a direct link between HAP deposition and advanced AMD (Szmacinski et al., 2020; Tan et al., 2018). Calcification within drusen can be visualized as "heterogeneous internal reflectivity within drusen" in AMD patients using multimodal imaging including optical coherence tomography (OCT) (Ouyang et al., 2013; Tan et al., 2018; Liu et al., 2022).

Thompson *et al.* proposed a novel mechanism for the growth and the initiation of sub-RPE deposits with implications for the development of AMD: "Sub-RPE deposit-growth, and maybe even formation, may be mediated by the formation of HAP shells on cholesterol-containing, naturally present extracellular lipid droplets at the RPE-choroid interface." (Thompson *et al.,* 2015). Accordingly, Arya et al. have shown that, in the presence of lipid droplets and homeostatic changes in calcium and phosphate availability in the sub-RPE-basal lamina space, HAP can precipitate on the surface of the lipid droplets. Then, on the surface of the HAP spherules drusen proteins can accumulate via directly interacting with HAP (Arya *et al.,* 2018). Altogether, these findings have led to the "meet, greet and stick" hypothesis by Bergen *et al.,* which proposes HAP crystals to act as a "seeding point for drusen formation". In this respect, Bergen *et al.* concluded that "this concept is novel [...] and may lead to HAP-based treatment strategies" (Bergen *et al.,* 2019).

Calcified drusen can be detected by multimodal imaging including optical coherence tomography (OCT) in 43% of patients with AMD (Liu *et al.,* 2022). Patients with this biomarker may be suitable candidates for therapeutic intervention aiming to resolve drusen calcification. Treatment efficacy can then be evaluated by monitoring drusen appearance and volume by multimodal imaging techniques, including OCT.

Apart from age-related macular degeneration, optic disc drusen (ODD) is a further disease where calcifications occur at an elastin rich layer named lamina cribrosa within the optic nerve head (Palmer *al* et., 2018). Optic disc drusen are calcified, acellular bodies, seen in the optic nerve head of up to 2% of the population. Although seldomly affecting visual acuity, visual field defects are common, and severe, ischemic complications causing irreversible vision loss are known to occur (Hamann *et al.,* 2018). The pathogenesis of ODD has not been fully elucidated, but dystrophic calcification of optic disc drusen has been attributed to their pathogenicity. One theory for the visual field loss is that calcified bodies compress adjacent ganglion cell axons, leading to ganglion cell death and axonal degeneration (Katz *et al.,* 2006). The origin of calcification in ODD is still not clearly understood. Interestingly, Raming *et al.* found an increased frequency of ODD in patients with PXE, a disease characterized by Bruch's membrane calcification. The association of a more extensive angioid streak length (an indicator of the state and extent of ocular Bruch's membrane calcification) and ODD suggested that ODD is associated with the degree of ectopic calcification, likely affecting the lamina cribrosa, an elastin rich area at the optic nerve head (Raming *et al.,* 2024). Conversely, it was proposed that resolution of dystrophic calcification in ODD may prevent ODD growth and complications arising from ODD (Bentin *et al.,* 2024).

Regarding possible methods to treat Bruch's membrane calcification Booij *et al.* in 2010 stated that "further elucidation of the calcification process (in Bruch's membrane) holds the promise that soft tissue and Bruch's membrane calcification can perhaps be influenced by drugs or by dietary means" (Booij *et al.,* 2010). Indeed, in this respect, LaRusso *et al.* were able to show that elevated dietary magnesium prevented connective tissue mineralization in a mouse model of PXE. In this paper, the authors however focused on the mineralization of the connective tissue capsule surrounding the vibrissae and not on BM calcifications (LaRusso *et al.,* 2009). Ethylenediaminetetraacetic acid (EDTA) chelation therapy has been used in ophthalmology to treat calcific band keratopathy (CBK), a chronic degenerative condition characterized by the deposition of greyish to whitish opacities in the superficial layer of the cornea, most frequently in the interpalpebral zone. CBK is referred to as primary or idiopathic. In the early stages, it remains asymptomatic; however, once it extends into the visual axis, it results in significant glare and visual disturbances. Various modalities have been used in the treatment of CBK, by far the most widely used method is EDTA chelation with the goal of removing calcium opacities and to restore a smooth ocular surface. Other methods include the use of a diamond burr, Nd:YAG laser, lamellar keratoplasty and phototherapeutic keratectomy. EDTA chelation therapy for CBK is performed at the slip lamp by preparing a 3.75% dilution of disodium EDTA in a tuberculin syringe. After the instillation of topical anesthesia, the epithelium overlying the band keratopathy is removed and a cellulose sponge or sterile cotton applicator is soaked in the diluted EDTA solution and rubbed against the calcium until its dissolution (Najjar *et al.,* 2004).

To our knowledge, EDTA or other chelating agents have so far not been used to treat Bruch's membrane or drusen calcifications. Intravitreal application of EDTA to rabbit eyes has been shown to be toxic to ocular structures when applied at a 0.1 molar concentration (which would equal 2900 $\mu g$ of EDTA) (Roll *et al.,* 1977).

Thus, a better form of delivering chelating agents to the calcified Bruch's membrane and the adjacent tissues, especially for the treatment of AMD and/or optic disc drusen is needed.

## SUMMARY OF INVENTION

This object is accomplished, inter alia, by a nanoparticle for use in a prophylaxis or in a treatment of age-related macular degeneration and/or optic nerve head drusen and a pharmaceutical composition of the independent claims.

The present invention provides in a first aspect a nanoparticle comprising a scaffold comprising a biodegradable material, an antibody, which is able to bind to a component of a Bruch's membrane, a component of a subretinal pigment epithelial deposit, or a component of an optic nerve head, and an anti-calcifying agent for use in a prophylaxis or in a treatment of age-related macular degeneration and/or optic nerve head drusen of an eye.

In one embodiment of the nanoparticle for use of the present invention, said prophylaxis or said treatment of age-related macular degeneration and/or optic nerve head drusen is accomplished by the prophylaxis or the treatment of calcified subretinal pigment epithelium deposit and/or calcified drusen of the eye.

In one embodiment of the nanoparticle for use of the present invention, said biodegradable material is human serum albumin.

In one embodiment of the nanoparticle for use of the present invention, said antibody, which is able to bind to the component of the Bruch's membrane, the component of the subretinal pigment epithelial deposit or the component of the optic nerve head, is an antibody targeted to the component of the Bruch's membrane, targeted to a shell protein of hydroxyapatite spherules in the subretinal pigment epithelial deposit, or targeted to the component of the optic nerve head, preferably the antibody is an anti-elastin antibody, an anti-fibronectin antibody, an anti-complement factor H antibody, an anti-beta-amyloid antibody or an anti-vitronectin antibody, more preferably the antibody, which is able to bind to the component of the Bruch's membrane, the component of the subretinal pigment epithelial deposit, or the component of the optic nerve head, is the anti-elastin antibody, and wherein the antibody, which is able to bind to the component of the Bruch's membrane, the component of the subretinal pigment epithelial deposit, or the component of the optic nerve head, is preferably covalently linked to the nanoparticle.

In one embodiment of the nanoparticle for use of the present invention, said anti-calcifying agent is a chelator or an inorganic pyrophosphate (PPi), or a sodium thiosulfate, preferably the chelator is diethylenetriaminepentaacetic acid (DTPA) or ethylenediaminetetraacetic acid (EDTA).

In one embodiment of the nanoparticle for use of the present invention, said nanoparticle has a negative surface charge.

In one embodiment of the nanoparticle for use of the present invention, said nanoparticle comprises a scaffold comprising human serum albumin, an anti-elastin antibody preferably covalently linked to the nanoparticle, and **DTPA** preferably covalently linked to the nanoparticle.

In a second aspect, the invention provides a pharmaceutical composition comprising the nanoparticle of the first aspect and one or more pharmaceutical acceptable excipient(s).

In one embodiment of the pharmaceutical composition of the present invention, said pharmaceutical composition is for use in a prophylaxis or in a treatment of age-related macular degeneration and/or optic nerve head drusen of an eye.

In one embodiment of the pharmaceutical composition for use of the present invention, said prophylaxis or treatment of age-related macular degeneration and/or optic nerve head drusen is accomplished by the prophylaxis or the treatment of calcified subretinal pigment epithelium deposit and/or calcified drusen of the eye.

In one embodiment of the nanoparticle for use, of the pharmaceutical composition, or of the pharmaceutical composition for use of the present invention, said nanoparticle or said pharmaceutical composition is administered intravenously, intravitreally, subconjunctivally, subtenonly, retrobulbarly, posteriorly, juxtasclerally, suprachoroideally, subretinally, or topically as eye drops.

**BRIEF DESCRIPTION OF FIGURES**

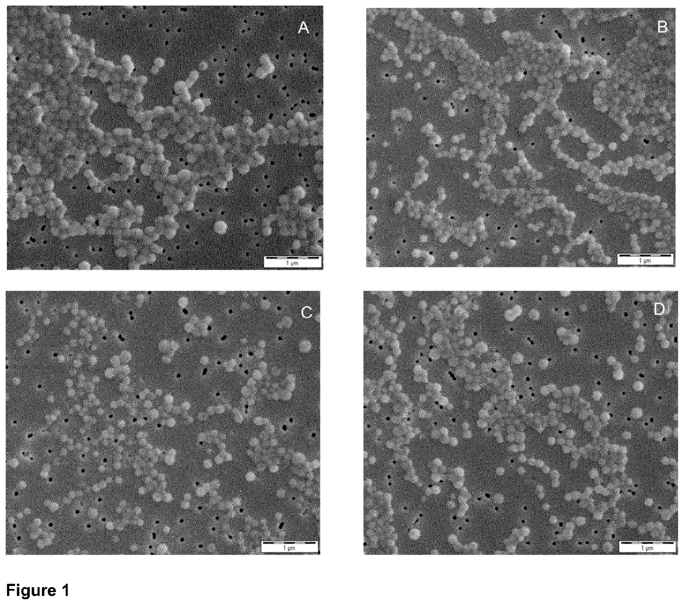

**Figure 1** Scanning electron microscopy images of HSA-NP (**A**), DTPA-(HSA-NP) (**B**), DTPA-(HSA-NP)-el ab (**C**), and DTPA-(HSA-NP)-IgG (**D**). The nanoparticles were prepared according to point 1.2.4 of the Materials and Methods section.

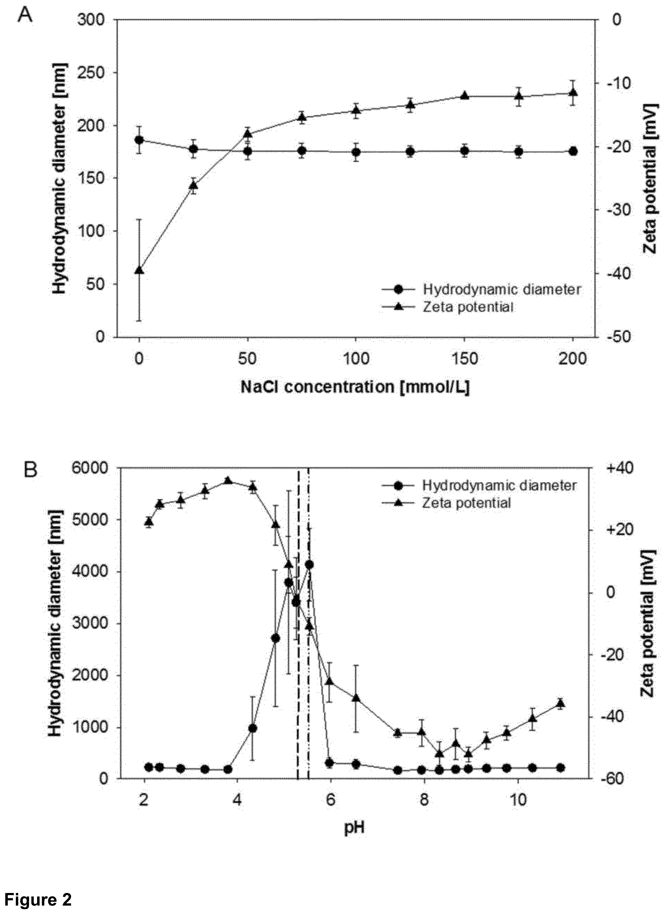

**Figure 2** The stability of DTPA-(HSA-NP) was evaluated over increasing electrolyte concentration (**A**) and a pH ranging from 2 to 11 (**B**) (mean $\pm$ SD, n = 3). Vertical lines in B represent the isoelectric points of HSA-NP (5.52 $\pm$ 0.07; -··-··-) and DTPA-(HSA-NP) (5.28 $\pm$ 0.11; - - -). The nanoparticles were prepared according to point 1.2.4 of the Materials and Methods section.

**Figure 3** Cell viability of (**A**) hCMEC/D3 cells, (**B**) Jurkat cells, and (**C**) primary macrophages and HUVECS after 24 h incubation with different NP formulations or pure DTPA (mean $\pm$ SD, n = 3). The nanoparticles were prepared according to point 1.2.4 of the Materials and Methods section.

**Figure 4** Cellular interaction of nanoparticles with hCMEC/D3 cells (**A**) and Jurkat cells (**B**) detected via live-cell imaging (mean $\pm$ SD, n = 3). The nanoparticles were prepared according to point 1.2.4 of the Materials and Methods section.

**Figure 5** Cellular interaction of nanoparticles with hCMEC/D3 cells (**A**) and Jurkat cells (**B**) measured via FACS analysis (mean $\pm$ SD, n = 3). The nanoparticles were prepared according to point 1.2.4 of the Materials and Methods section.

**Figure 6** Visualization of cellular interaction of different NP formulations with hCMEC/D3 cells after 24 h incubation. NP are visualized by red fluorescence of conjugated PF633P, cell nuclei were stained with DAPI (blue) and membranes with WGA Alexa Fluor® 350 (blue). **A:** pHSA-NP; **B:** (pHSA-NP)-el ab; **C:** (pHSA-NP)-IgG; **D:** DTPA-(pHSA-NP); **E:** DTPA-(pHSA-NP)-el ab; **F:** DTPA-(pHSA-NP)-IgG. The nanoparticles were prepared according to point 1.2.4 of the Materials and Methods section.

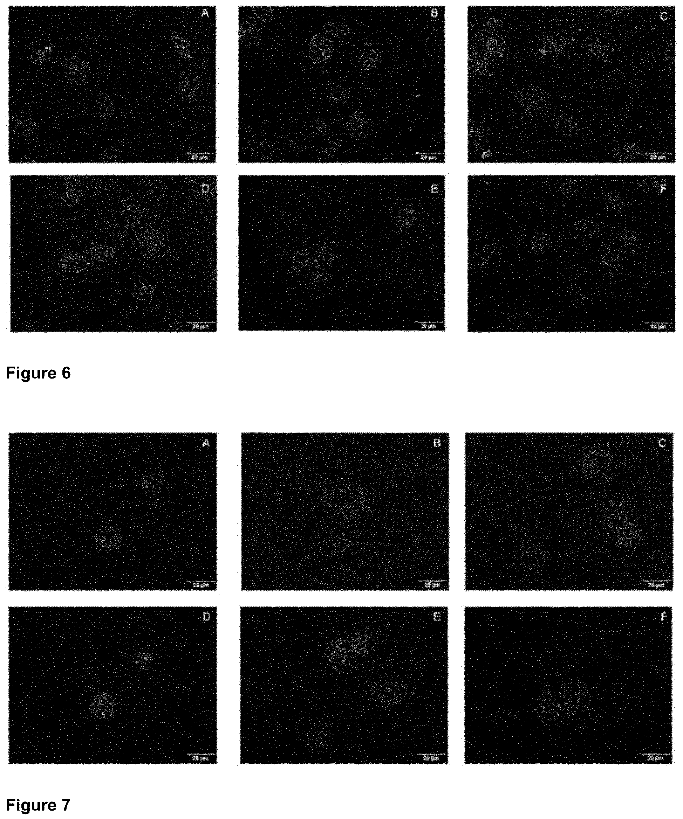

**Figure 7** Visualization of cellular interaction of different NP formulations with Jurkat cells after 24 h incubation. NP are visualized by red fluorescence of conjugated PF633P, cell nuclei were stained with DAPI (blue) and membranes with WGA Alexa Fluor® 350 (blue). **A:** pHSA-NP; **B:** (pHSA-NP)-el ab; **C:** (pHSA-NP)-IgG; **D:** DTPA-(pHSA-NP); **E:** DTPA-(pHSA-NP)-el ab; **F:** DTPA-(pHSA-NP)-IgG. The nanoparticles were prepared according to point 1.2.4 of the Materials and Methods section.

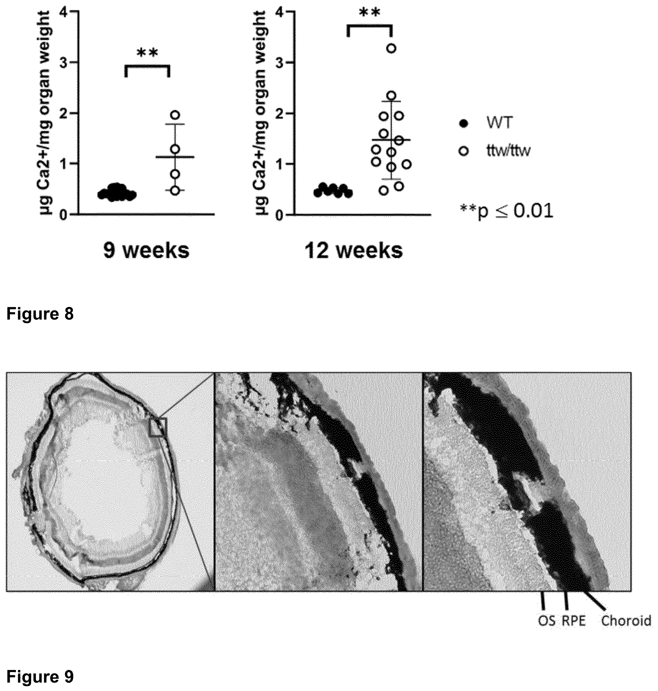

**Figure 8** Quantification of calcification of ocular tissue in ttw/ttw mice on a diet containing high phosphorus and low magnesium compared to wild-type mice on normal chow diet.

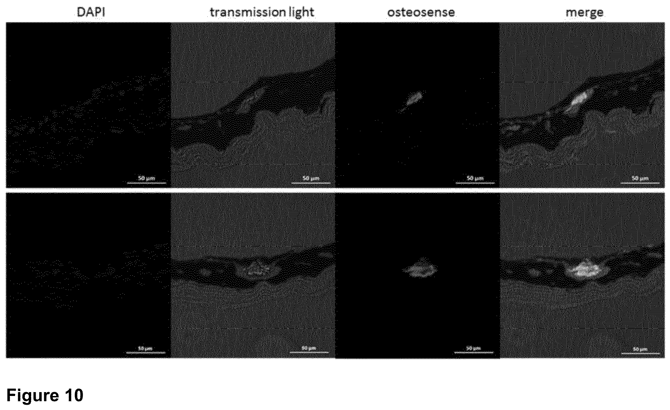

**Figure 9** Drusenoid calcification at the choroid- RPE interface in ttw/ttw mouse (on a high phosphate ("acceleration") diet) at 12 weeks of age. Middle panel and right panel show higher magnifications of insert of left panel. Alizarin Red S staining, cross section. OS: outer segment, RPE: retinal pigment epithelium. Choriod: Choriocapillaris **Figure 10** Drusenoid calcification at the RPE-choroid interface in ttw/ttw mouse at 12 weeks of age (on acceleration diet). Cross section of flat mount sample, posterior segment of the eye, Osteosense staining. Flat mounted sample was stained after careful removal of photoreceptor layer and of the RPE.

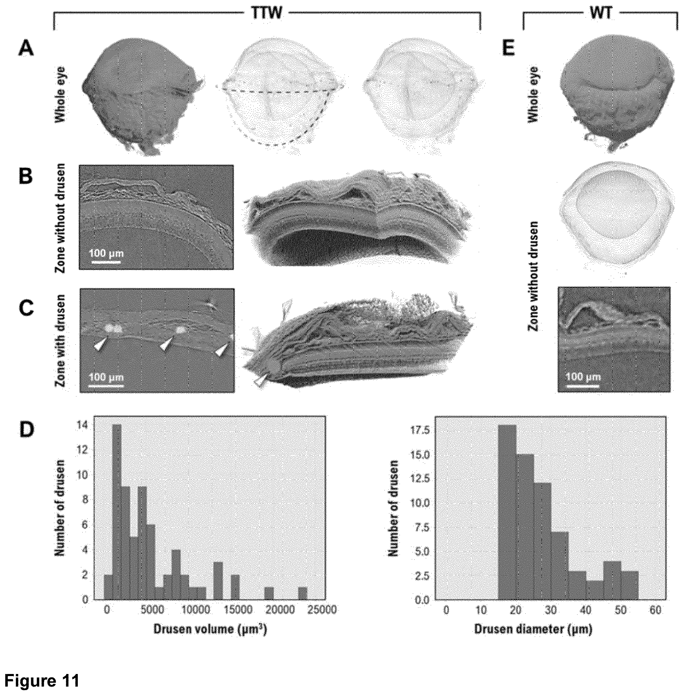

**Figure 11** X-ray microscopy of drusenoid bodies in the retina of ttw/ttw mice on high phosphate diet. (**A**) Examination of the mineral in the full eye of ttw/ttw mice. Segmentation of microscale mineral deposits follow two dominant arcs, wrapping around the ciliary margin like a string of pearls and extending back towards the macula. (**B**) Higher-magnification view of unmineralized retina, where layers of the retina are clearly stratified. (**C**) Higher-magnification view of mineralized portions of the retina (arrowheads) with microscale mineral deposits situated beneath the choroid, roughly localized to Bruch's membrane and the retinal pigment epithelium. (**D**) Morphometry of the drusenoid deposits across the whole ttw/ttw eye. The median calcified deposit has a volume of 4200 $\mu m^3$ and diameter of 25 $\mu m$. (**E**) X-ray microscopy scans of the full WT eye and high-magnification volume of the retina. No clear drusenoid features are present in the WT eye.

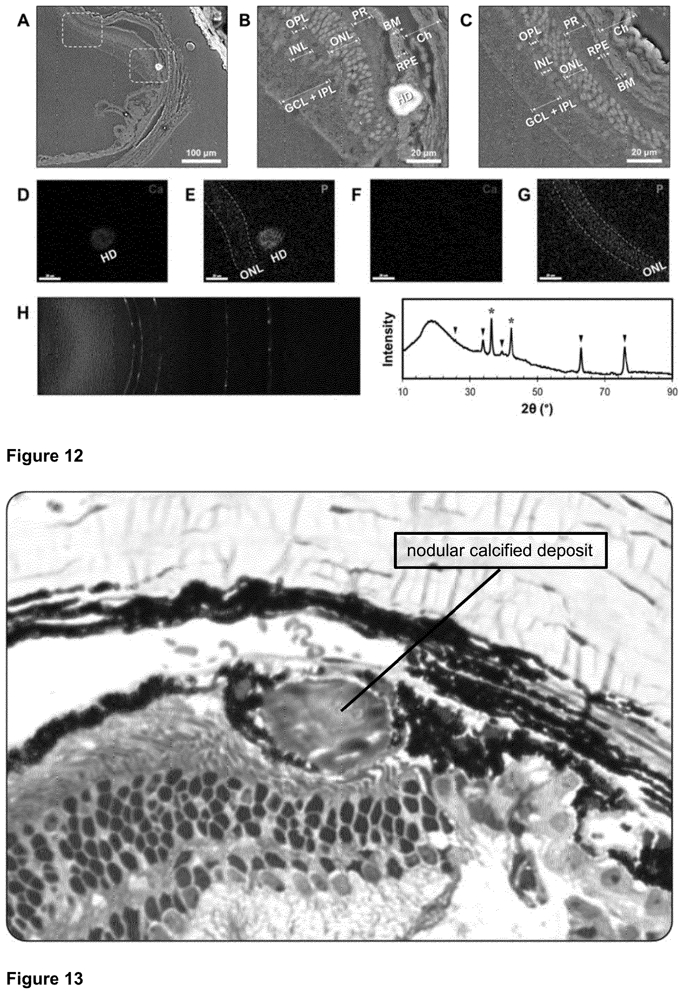

**Figure 12** Mineral characterization of drusenoid bodies in ttw/ttw retina. (**A-C**) Scanning electron microscopy using backscattered electron imaging in the posterior segment of an unstained retina. (**B**) Region of retina containing a drusenoid body. The drusenoid body is located outside of the photoreceptors, disrupting the continuity of both the retinal pigment epithelium and Bruch's membrane. (**C**) Region of the same eye without drusenoid bodies. (**D-G**) Energy-dispersive X-ray spectroscopy of the retina, with and without drusenoid bodies. Strong calcium and phosphorus signals are localized to the drusenoid body. (**H**) 2D and 1D X-ray diffraction spectra of the drusenoid body. The drusenoid body appears polycrystalline in nature, with diffraction peaks corresponding to an apatite phase (arrowheads) and an unidentified mineral phase, possibly minor peaks of whitlockite or brushite (asterisks). GCL: Ganglion Cell Layer; IPL: Inner Plexiform Layer; INL: Inner Nuclear Layer; OPL: Outer Plexiform Layer; ONL: Outer Nuclear Layer; PR: Photoreceptors; RPE: Retinal Pigment Epithelium; HD: Hard Drusen/calcified deposit; BM: Bruch's Membrane; Ch: Choroid

**Figure 13** Light microscopy image of a drusenoid nodular calcified deposit near the ora serrata. Mineralized deposit lies between the choroid and the RPE (Toluidine blue staining).

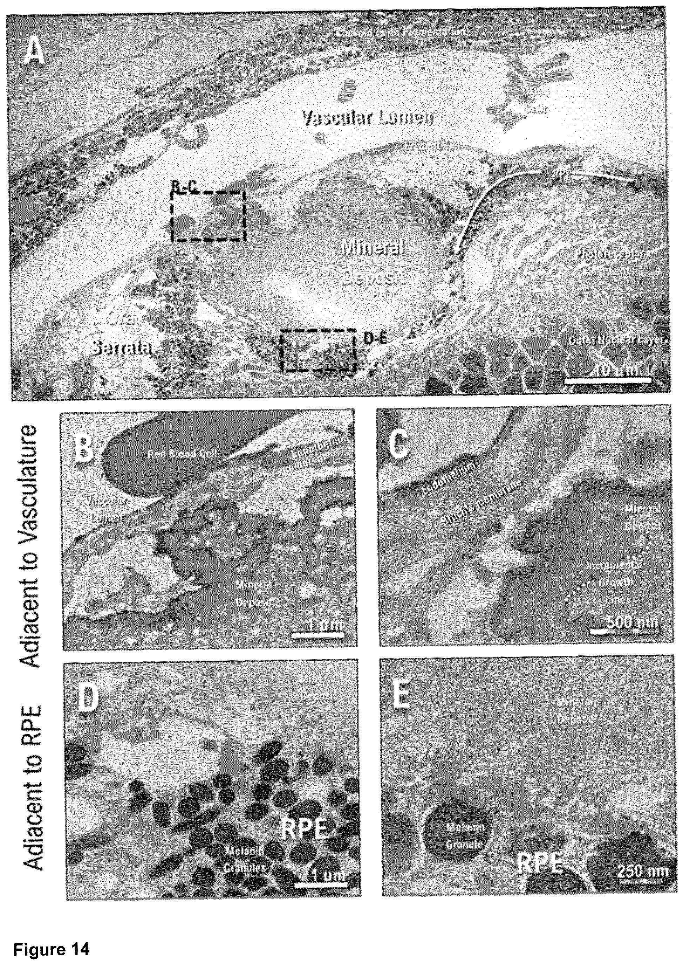

**Figure 14** Transmission Electron Microscopy images of mineral deposit near the ora serrata. (**A**) Mineral deposit lies in the retina (near the ora serrata), outside of a vessel, separating the RPE from Bruch's membrane. (**B, C**) Mineral deposit approaches the endothelium of the nearby capillary, but does not actually penetrate to the lumen. (**C**) The mineral deposit does not appear to be a homogenous mass, seemingly containing some globular organic and incremental lines where mineralization ceases/resumes. (**D, E**) Mineral deposit slightly distorts the RPE, and potentially the nearby photoreceptor segments. (Scattered boxes in (**A**) refer to images in higher magnification (**B-E**))

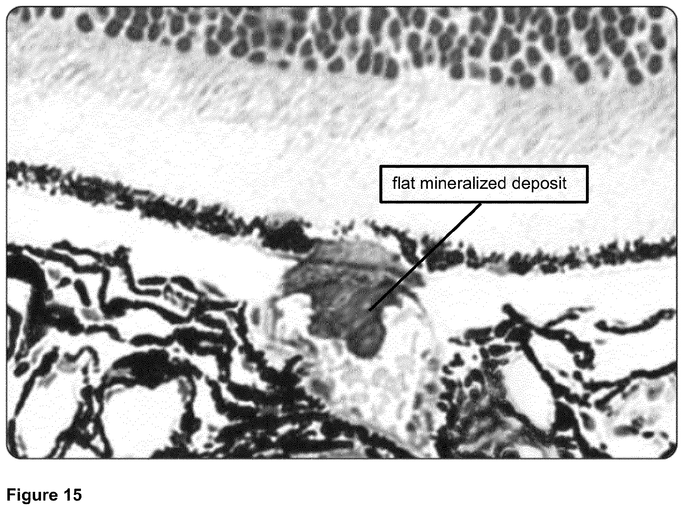

**Figure 15** Light microscopy image of a flat mineralized deposit along posteriorly oriented arc. The mineralized deposit occupies most of the space between the choriocapillaris and the RPE (Toluidine blue staining).

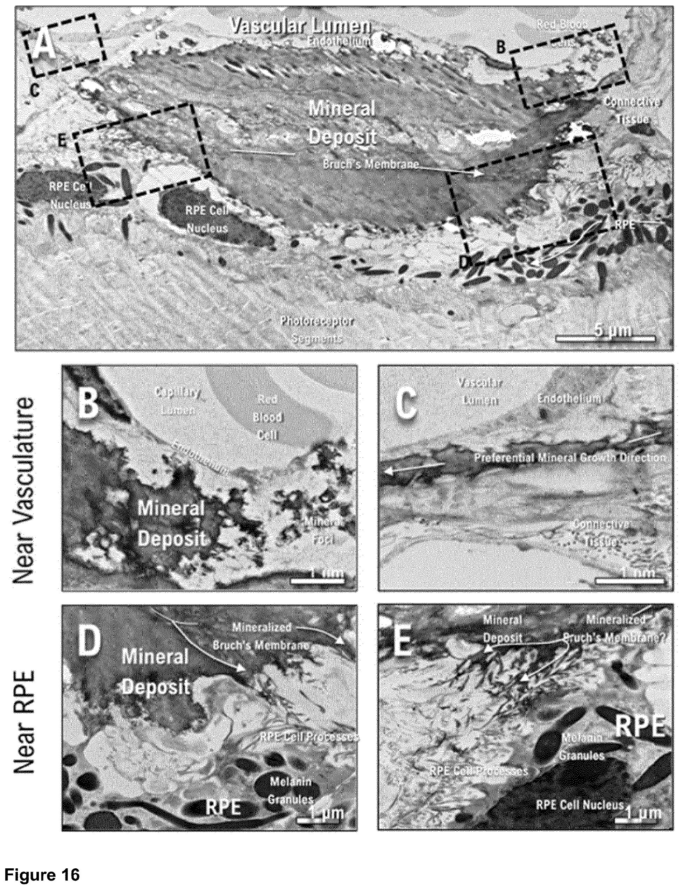

**Figure 16** Transmission Electron Microscopy images of flat mineralized deposit localized along the posteriorly oriented arc. (**A**) Mineral deposit approaches the endothelium of the nearby capillary, but does not actually penetrate to the lumen. Mineral deposit slightly distorts the RPE, and there appears to be fewer melanin granules in the RPE beneath the deposit (or loss of cells). (**B**) The formation of new mineral foci may partly take place around globular organic masses, forming the 'ring' shapes of mineral. (**C**) Preferential propagation of the mineral deposit may also be along the loose connective tissue surrounding the capillary, encapsulating the BM along the way. (**D, E**) The BM is presumably enveloped within the mineral deposit, seemingly some texture in the approximate site of the BM that could be mineralized collagen. (Scattered boxes in (**A**) refer to images in larger magnification (**B-E**))

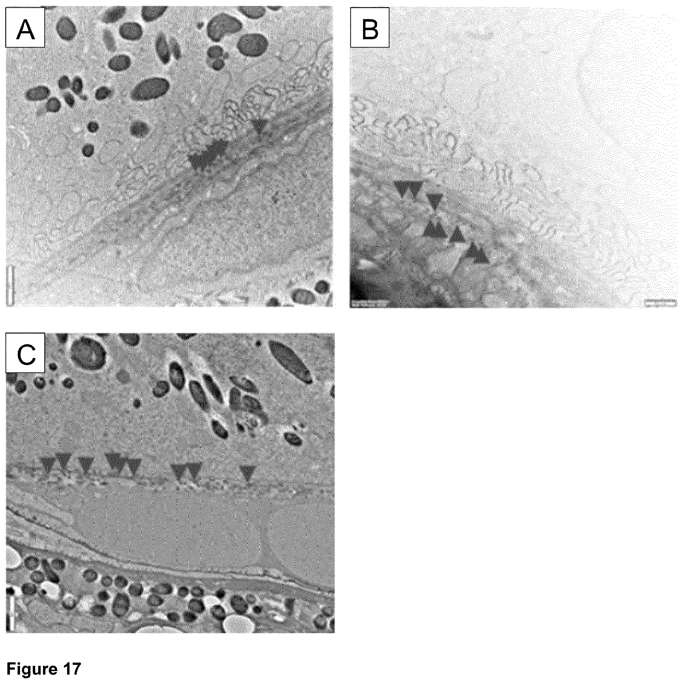

**Figure 17** Localization of anti-elastin coupled magnetite containing HSA-based NP in Bruch's membrane in ttw/ttw mice in vivo. Anti-elastin coupled HSA-based NP were coupled to magnetite for better visualization and injected into the tail veins of 10 week old ttw/ttw mice (on acceleration diet). Mice were sacrificed 4 hours or 48 hours after injection, and enucleated eyes were examined by TEM. (**A, B**) 4 hours after injection, (**C**) 48 hours after injection. (**A, C**) Scale: 1.0 $\mu$m. (**B**), Scale 600 nm. Arrow heads point to the nanoparticles. The nanoparticles were prepared according to point 1.2.6 of the Materials and Methods section.

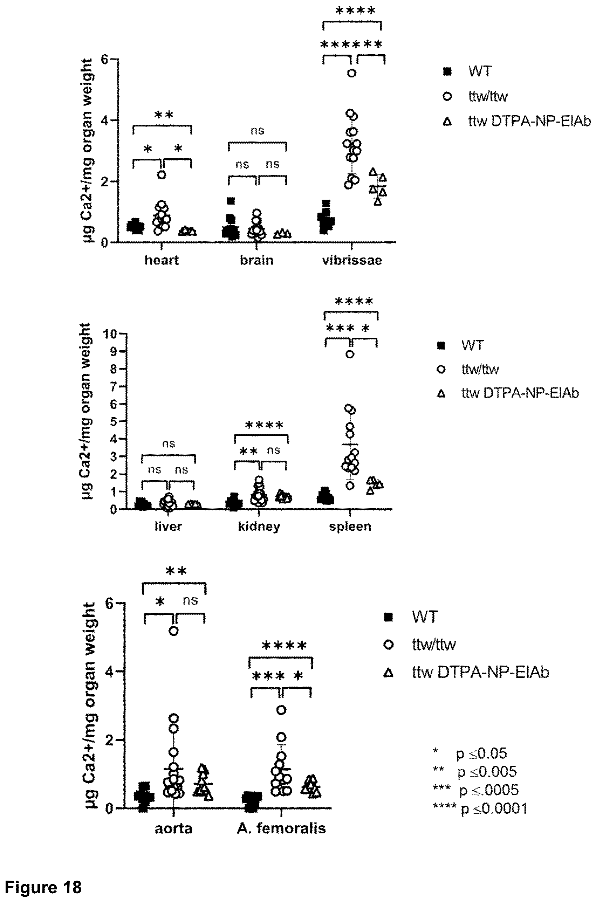

**Figure 18** Dissolution of calcifications in different organ tissues of ttw/ttw mice. DTPA bound HSA-based NP containing an anti-elastin antibody were injected into the tail vein of ttw/ttw mice on an acceleration diet. Injections started at the age of 10 weeks at a dose of 20 mg/kg and continued twice weekly for two weeks. Mice were sacrificed at the age of 12 weeks. Calcium content of organs from treated ttw/ttw mice was compared to untreated ttw/ttw mice and to wild-type mice of the same age. WT: wild type mice; ttw/ttw: Enpp1-deficient ttw/ttw mice on acceleration diet; ttw DTPA-NP-ElAb: ttw/ttw mice on acceleration diet treated with NP. The nanoparticles were prepared according to point 1.2.5 of the Materials and Methods section.

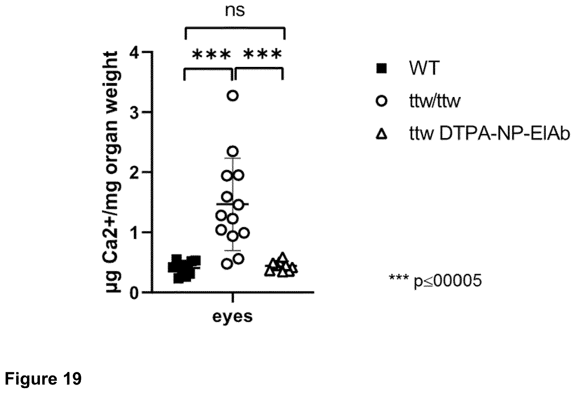

**Figure 19** Dissolution of ocular calcifications in ttw/ttw mice. DTPA bound HSA-based NP containing an anti-elastin antibody were injected into the tail vein of ttw/ttw mice on acceleration diet. Injections started at the age of 10 weeks at a dose of 20 mg/kg body weight and were repeated twice weekly for two weeks (4 injections in total). Mice were sacrificed at the age of 12 weeks. Calcium content of eyes from treated ttw/ttw mice was compared to eyes from untreated ttw/ttw mice and to eyes from wild-type mice of the same age. WT: wild type mice; ttw/ttw: Enpp1-deficient ttw/ttw mice on acceleration diet; ttw DTPA-NP-ElAb: ttw/ttw mice on acceleration diet treated with NP. The nanoparticles were prepared according to point 1.2.5 of the Materials and Methods section.

## DETAILED DESCRIPTION OF THE INVENTION

A ttw/ttw mouse (tiptoe walking mouse) (or TTW used synonymously) used herein as animal model has non-physiological calcifications, e.g. of an Achilles tendon, hair follicles of whiskers, and aorta (Hosoda *et al.,* 1981). It was shown that this ectopic calcification is due to a point mutation of a *NPPS* gene encoding a nucleotide pyrophosphatase (Npps), resulting in a non-functional truncated protein (Okawa *et al.,* 1998). This Npps is an ectonucleotide pyrophosphatase/phosphodiesterase 1 (ENPP1). Thus, the ttw/ttw mouse has an ENPP1 deficiency and further has cartilage calcification (Bertrand *et al.,* 2012). In humans, ENPP1 deficiency leads to generalized arterial calcification of infancy (Bäck *et al.,* 2019). There are further mouse models showing calcification of Bruch's membrane (BM), but to our knowledge no calcified subretinal pigment epithelium deposit or calcified drusen typical for age-related macular degeneration (AMD) or optic nerve head drusen were shown in these models. In one of these mouse models the *ENPP1* gene was knocked-out, however at a different point within the DNA sequence of the *ENPP1* gene compared to the ttw/ttw mouse. This knock-out results in functional changes in the retina and calcification of Bruch's membrane, but subretinal pigment epithelium deposits or calcified drusen were not reported (Lengyel *et al.,* 2022). In an Abcc6 deficient mouse model of Pseudoxanthoma elasticum disease calcification of Bruch's membrane was detected, but no subretinal pigment epithelium deposit or calcified drusen were reported (Gorgels *et al.,* 2012).

The inventors showed that ttw/ttw mice aged 9 to 12 weeks on a phosphate rich ("acceleration") diet have calcifications of the ocular tissue (Figure 8), e.g. at Bruch's membrane and adjacent tissues (shown in Example 5). This was a surprising result, since ttw/ttw mice on normal diet, as used in Keuth *et al.,* 2020, would not reach an age when ocular calcification could be expected based on the literature (Lengyel *et al.* 2022, Gorgels *et al.* 2012) due to serious health problems that raise animal welfare concerns.

The inventors showed that ttw/ttw mice aged 9 to 12 weeks on an acceleration diet have drusenoid calcification at the retinal pigment epithelium (RPE) -choroid interface (Figures 8, 9, 10). Using X-ray microscopy, they found a segmentation of microscale mineral deposits following two dominant arcs, wrapping around the ciliary margin like a string of pearls and extending back towards the macula (Figures 11 - 16).

It was further demonstrated, that these drusen or drusenoid structures appear polycrystalline in nature, with diffraction peaks corresponding to an apatite phase (arrowheads) and an unidentified mineral phase (Figure 12).

The inventors demonstrated that a nanoparticle comprising human serum albumin (HSA) as scaffold, an anti-elastin antibody, and magnetite accumulates in Bruch's membrane of ttw/ttw mice after intravenous injection (shown in Example 8 and Figure 17).

Furthermore, the inventors succeeded in reducing the ocular calcification, including the calcified drusen and calcified drusenoid bodies, in ttw/ttw mice on an accelerated diet using a nanoparticle comprising human serum albumin (HSA), an anti-elastin antibody, and a diethylenetriaminepentaacetic acid (DTPA) administered intravenously (shown in Example 9, Figure 19).

Thus, the inventors surprisingly found that the nanoparticle having the scaffold comprising a biodegradable material and further comprising the antibody targeting (or able to bind to or targeted to) the Bruch's membrane, a component of the subretinal pigment epithelial deposit of an eye, or a component of an optic nerve head and an anti-calcifying agent is able to reduce ocular calcifications including the calcified drusen and calcified drusenoid bodies. Thus, this nanoparticle could be used for prophylaxis or treatment of age-related macular degeneration (AMD) and/or optic nerve head drusen of the eye, since calcified drusen and calcified drusenoid bodies are symptoms of AMD and/or optic nerve head drusen.

The present invention provides therefore in a first aspect the nanoparticle comprising the scaffold comprising the biodegradable material, the antibody, which is able to bind to a component of the Bruch's membrane, the component of the subretinal pigment epithelial deposit, or the component of the optic nerve head, and the anti-calcifying agent for use in the prophylaxis or in the treatment of age-related macular degeneration and/or optic nerve head drusen of an eye.

It is preferred that the prophylaxis or the treatment of age-related macular degeneration and/or optic nerve head drusen is accomplished by the prophylaxis or the treatment of calcified subretinal pigment epithelium deposit and/or calcified drusen of the eye.

This invention comprises the knowledge of the importance of calcified subretinal pigment epithelium deposit and/or calcified drusen for age-related macular degeneration (AMD) and for optic nerve head drusen resulting from the deposition of e.g. calcium ions, for which the inventors established a suitable mouse model, and the knowledge of the anatomical situation of Bruch's membrane and adjacent tissues. With the inventive use of the nanoparticle, the inventors at first use the possibility of the anti-calcifying agent that binds the ions, e.g. calcium ions, which are one causative for the calcification, and second to use a specific targeting, facilitated by the antibody specific for the component of Bruch's membrane, the component of the subretinal pigment epithelial deposit, or the component of the optic nerve head and combining these uses with the nanoparticle comprising the biodegradable material. This results in a biocompatible nanoparticle, which is equipped with the antibody, for specifically binding to the Bruch's membrane, the subretinal pigment epithelial deposit, or the component of the optic nerve head, and the anti-calcifying agent. Since the nanoparticle is targeted to the component of Bruch's membrane, the subretinal pigment epithelial deposit, or the component of the optic nerve head this nanoparticle is predestinated for the use in the prophylaxis and treatment of age-related macular degeneration and/or optic nerve head drusen, e.g. the calcification of the subretinal pigment epithelium deposit and/or the drusen.

The nanoparticle for the inventive use comprises the scaffold comprising the biodegradable material. Therefore, the nanoparticle could be degraded by enzymes of the human body without an unwanted deposit of a component of the material. Biodegradable materials usable for *in vivo* administration are known to the skilled person and could be found in pharmacopoeias or publications of a responsible pharmaceutical regulatory authority, like the FDA. Human serum albumin (HSA) is herein preferred as biodegradable material. HSA could be degraded in the human body and found to be safe by pharmaceutical regulatory authorities. Thus, HSA is ideal as biodegradable material used as the scaffold of the nanoparticle.

The nanoparticle for the inventive use further comprises the antibody, which is able to bind or is directed to the component of the Bruch's membrane, the component of the subretinal pigment epithelial deposit, or the component of the optic nerve head. Thus, the antibody, and therefore also the nanoparticle to which the antibody is bound, is specifically targeted to the component of the Bruch's membrane, the component of the subretinal pigment epithelial deposit, or the component of the optic nerve head. Since the antibody is able to specifically bind his cognate antigen, the antibody (and the nanoparticle bound thereto) is specifically directed to the component of the Bruch's membrane, the component of the subretinal pigment epithelial deposit, or the component of the optic nerve head, the antigen of the antibody. Thus, the antibody, which is able to bind to the component of the Bruch's membrane, the component of the subretinal pigment epithelial deposit, or the component of the optic nerve head is the antibody targeted to or directed to the component of the Bruch's membrane or targeted to a shell protein of hydroxyapatite spherules in the subretinal pigment epithelial deposit or targeted to the component of the optic nerve head. In general, any component of Bruch's membrane, of the subretinal pigment epithelial deposit, or of the component of the optic nerve head could be a suitable target or antigen of the antibody used herein. Preferably the targeted component is chosen from components mostly found in Bruch's membrane, in the subretinal pigment epithelial deposit, or a lamina cribrosa of the optic nerve head drusen in a suitable amount to circumvent binding of the antibody to its targeted component outside of Bruch's membrane, of the subretinal pigment epithelial deposit, or of the component of the optic nerve head. Suitable components of the Bruch's membrane, which could serve as suitable target or antigen of the antibody are elastin, fibronectin, vitronectin, collagen VI and other proteins of the Bruch's membrane. Suitable components of the subretinal pigment epithelial deposit, which could serve as suitable target or antigen of the antibody are vitronectin, factor H and beta-amyloid, which are preferred antigens/targets of the antibody coupled to the nanoparticle for the inventive use. A suitable component of the lamina cribosa of the optic nerve head is elastin, which could serve as a suitable target or antigen of the antibody. The more preferred target of the antibody is elastin, which is the main component of the elastic layer of Bruch's membrane and the component of the lamina cribosa of the optic nerve head. Thus, preferably the antibody coupled to the nanoparticle for the inventive use is then anti-elastin antibody, an anti-fibronectin antibody, an anti-complement factor H antibody, an anti-beta-amyloid antibody or an anti-vitronectin antibody, more preferably the antibody is the anti-elastin antibody. Furthermore, the antibody is preferently covalently bound to the nanoparticle.

The term "antibody" as used herein and in the context of the present invention may comprise chimeric antibodies, humanized antibodies, monovalent antibodies, polyvalent antibodies, low-molecular antibodies, a diabody, a Fab fragment, or a scFv.

Chimeric antibodies refer to antibodies comprising variable and constant regions of different origins ligated with each other. For example, mouse-human heterogeneous chimeric antibodies are antibodies comprising the heavy and light chain variable regions of a mouse antibody and the heavy and light chain constant regions of a human antibody. Mouse antibody variable region-encoding DNAs are ligated with human antibody constant region-encoding DNAs, and the ligation products can be incorporated into expression vectors to prepare chimeric antibody-expressing recombinant vectors. Cells transformed with these vectors (recombinant cells) can be cultured for the expression of the DNA insert to obtain the chimeric antibodies produced during the culture.

In general, the chimeric antibodies comprise non-human animal-derived antibody variable regions and human antibody-derived constant regions. By contrast, the humanized antibodies comprise non-human animal-derived antibody complementarity-determining regions (CDRs), human antibody-derived framework regions (FRs), and human antibody-derived constant regions. The humanized antibodies are also called reshaped human antibodies. Specifically, for example, humanized antibodies comprising non-human animal (e.g., mouse) antibody CDRs grafted in human antibodies are known in the art. The humanized antibodies are useful as active ingredients for a therapeutic agent of the present invention, owing to their reduced antigenicity in the human body.

Each antibody variable region usually comprises 3 CDRs flanked by 4 FRs. The CDR regions substantially determine the binding specificity of the antibody. The CDRs have diverse amino acid sequences. On the other hand, amino acid sequences constituting the FRs often exhibit high homology among antibodies having different binding specificities. Therefore, in general, the binding specificity of a certain antibody can allegedly be transplanted to other antibodies through CDR grafting.

The antibody bound to the nanoparticle and present in a pharmaceutical composition of the present invention may encompass bivalent antibodies typified by IgG (IgG1, IgG2, IgG4, etc.) and also monovalent antibodies or polyvalent antibodies typified by IgM as long as these antibodies bind to the component of Bruch's membrane, to the component of the subretinal epithelial deposit, or to the component of the optic nerve head. The polyvalent antibody that may be present in the pharmaceutical composition of the present invention may encompass polyvalent antibodies having antigen-binding sites, all of which are the same as each other or some or all of which are different from each other.

The antibody bound to the nanoparticle and present in the pharmaceutical composition of the present invention may also be a low-molecular antibody that encompasses an antibody fragment deficient in a portion of the whole antibody (e.g., whole IgG). Such partial deficiency of the antibody molecule is accepted as long as the resultant antibody fragment is capable of binding to the component of Bruch's membrane, to the component of the subretinal epithelial deposit, or to the component of the optic nerve head. It is preferred that the antibody fragment employed in the pharmaceutical composition according to the present invention should contain one or both of heavy chain variable (VH) and light chain variable (VL) regions. It is also preferred that the antibody fragment employed in the pharmaceutical composition according to the present invention should contain CDRs. The number of CDRs contained in the antibody fragment employed in the pharmaceutical composition of the present invention is not particularly limited and is preferably at least 6 CDRs: heavy chain CDR1, CDR2, and CDR3 and light chain CDR1, CDR2, and CDR3.

The term "diabody" as used herein and in the context of the present invention may refer to a bivalent antibody fragment constructed by gene fusion. The diabody is a dimer comprising two polypeptide chains. Usually, each of the polypeptide chains constituting the dimer comprises heavy and light chain variable regions linked via a linker on the same chain. The linker in the diabody is generally too short to allow paring between heavy and light chain variable regions on the same chain. Specifically, the number of amino acid residues constituting the linker is, for example, approximately 5 residues. Therefore, heavy and light chain variable regions encoded on the same polypeptide chain cannot together form a single chain variable region fragment. Instead, they form a dimer by pairing with another single chain variable region fragment. As a result, the diabody has two antigen-binding sites.

The scFv, as used in the context of the present invention, may be obtained by linking heavy and light chain variable regions of the antibody. In the scFv, the heavy and light chain variable regions are linked via a linker, preferably, a peptide linker. The heavy and light chain variable regions in the scFv can be derived from any of the antibodies described in the present specification. The peptide linker that links the variable regions is not particularly limited. For example, an arbitrary single chain peptide of approximately 3 to 25 residues can be used as the linker.

The nanoparticle for the inventive use further comprises the anti-calcifying agent. The anti-calcifying agent or anti-calcifying drug (both terms are used synonymously herein) is able to resolve the calcification of the subretinal pigment epithelium deposit and/or drusen. The term anti-calcifying drug or agent is known to the skilled person. Ideally this drug could bind the ions, e.g. calcium ions, responsible for the calcification such that the nanoparticle, to which the drug is bound, could also be used in the prophylaxis of the calcification. Such anti-calcifying drug may be a chelator, which is able to bind divalent ions, like $Ca^{2+}$. Suitable chelators are known to the skilled person. Preferably diethylenetriaminepentaacetic acid (DTPA) or ethylenediaminetetraacetic acid (EDTA) are used as chelator and anti-calcifying drug. Further preferred anti-calcifying drugs are inorganic pyrophosphate (PPi) and sodium thiosulfate. Especially preferred as anti-calcifying drug is diethylenetriaminepentaacetic acid (DTPA). The anti-calcifying drug could be covalently bound to the nanoparticle.

The nanoparticle used in the invention has preferably a negative surface charge. The surface charge could be expressed as zeta potential. The zeta potential is measured in that a part of the nanoparticle containing suspension is transferred in a special capillary cuvette. Using laser Doppler microelectrophoresis, the electrical mobility of the particles was measured on the Zetasizer Nano ZS (Malvern Instruments GmbH, Herrenberg, Germany) in zeta potential mode at an applied voltage of 140 V and a temperature of 22 °C. After evaluation with the Zetasizer device software, this velocity was transferred to the zeta potential output (e.g. described in Keuth *et al.,* 2020).

Thus, the especially preferred nanoparticle for use in the prophylaxis or treatment of the calcification of age-related macular degeneration and/or optic nerve head drusen comprises the scaffold comprising human serum albumin, the anti-elastin antibody preferably covalently linked to the nanoparticle, and DTPA preferably covalently linked to the nanoparticle. The preferred nanoparticle has the zeta potential of about -35.4 mV measured as described below and a hydrodynamic diameter of 179.8 nm. The hydrodynamic diameter is measured in that an aliquot of 10 μl of the purified particle suspension is diluted in a polystyrene cuvette with 2 ml ultrapure water to ensure free diffusion movement of the nanoparticles. The samples are measured with the Zetasizer Nano ZS (Malvern Instruments GmbH, Herrenberg, Germany) at a temperature of 22 °C and a backscattering angle of 173°. The mean hydrodynamic diameter and the intensity-weighted particle size distribution are calculated using the corresponding Zetasizer software (e.g. described in Keuth *et al.,* 2020). Thus, the preferred nanoparticle is therefore suitable for intravitreal application due to its negative surface charge (cf. Kim *et al.,* 2009). Furthermore, the nanoparticle used in the *in-vivo* experiments has also the right size for taken up by Bruch's membrane after intravenous application. It was shown that nanoparticles with a diameter ranging from 270 nm to 420 nm could be delivered to laser treated eyes via intravenous injection resulting in that the healthy eye does not uptake the nanoparticle (cf. Singh *et al.,* 2009). Further, the preferred nanoparticle is specifically targeted to the component of Bruch's membrane, to the component of the subretinal pigment epithelial deposit, or to the component of the optic nerve head due to the antibody coupled to the nanoparticle. This may result in lower side effects of the administered nanoparticle.

In a second aspect, the invention provides a pharmaceutical composition comprising the nanoparticle mentioned above and one or more pharmaceutical acceptable excipient(s).

In one embodiment, said pharmaceutical composition is for use in the prophylaxis or in the treatment of age-related macular degeneration and/or optic nerve head drusen of the eye.

It is preferred that said prophylaxis or treatment of age-related macular degeneration and/or optic nerve head drusen mentioned before is accomplished by the prophylaxis or the treatment of calcified subretinal pigment epithelium deposit and/or calcified drusen of the eye.

The nanoparticle for the inventive use, the pharmaceutical composition or the pharmaceutical composition for the inventive use could be administered via intravenous injection, intravitreal injection, subconjunctival injection, subtenon injection, retrobulbar injection, posterior juxtascleral injection, suprachoroideal or subretinal injection, or topical application as eye drops. Because of their negative surface charge, the nanoparticle is suited for intravitreal injection, since positive surface charged particles would not reach the posterior part of the eye (cf. Kim *et al.,* 2009).

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "and/or", wherever used herein, includes the meaning of "and", "or" and "all or any other combination of the elements connected by said term".

The terms "less than" or in turn "more than" do not include the concrete number. For example, "less than 20" means less than the number indicated. Similarly, "more than" or "greater than" means more than or greater than the indicated number, e.g. "more than 80%" means more than or greater than the indicated number of 80%.

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step. When used herein the term "comprising" can be substituted with the term "containing" or "including" or sometimes, when used herein, with the term "having". When used herein, "consisting of" excludes any element, step, or ingredient not specified.

The term "including" means "including but not limited to", "Including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments, only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

The invention may, in addition to the claims, be characterized by the following items:

1. A nanoparticle comprising a scaffold comprising a biodegradable material, an antibody targeting a component of a Bruch's membrane, and an agent for use in a prophylaxis or in a treatment of a pathological change of Bruch's membrane and/or an adjacent tissue, including a retinal pigment epithelium and a choroid of an eye.

2. The nanoparticle for use of item 1, wherein the agent is an anti-calcifying agent and wherein the pathological change is a calcification of Bruch's membrane and/or the adjacent tissue, including the retinal pigment epithelium and the choroid of the eye.

3. The nanoparticle for use of any one of the preceding items, wherein the biodegradable material is human serum albumin.

4. The nanoparticle for use of any one of the preceding items, wherein the antibody is an anti-elastin antibody or an anti-vitronectin antibody and wherein the antibody is preferably covalently linked to the nanoparticle.

5. The nanoparticle for use of any one of the preceding items, wherein the agent is a chelator, preferably diethylenetriaminepentaacetic acid (DTPA) or ethylenediaminetetraacetic acid (EDTA), inorganic pyrophosphate (PPi), or sodium thiosulfate.

6. The nanoparticle for use of any one of the preceding items, wherein the nanoparticle has a negative surface charge.

7. The nanoparticle for use of any one of the preceding items, wherein the nanoparticle comprises the scaffold comprising human serum albumin, the anti-elastin antibody preferably covalently linked to the nanoparticle, and DTPA preferably covalently linked to the nanoparticle.

8. A pharmaceutical composition comprising the nanoparticle of any one of the preceding items and pharmaceutical acceptable excipients for use in a prophylaxis or in a treatment of a pathological change of Bruch's membrane and/or adjacent tissues, including a retinal pigment epithelium and a choroid of an eye.

9. The pharmaceutical composition for use of item 8, wherein the pathological change is a calcification of Bruch's membrane and/or an adjacent tissue, including a retinal pigment epithelium and a choroid of an eye.

10. The nanoparticle for use of any one of items 1 to 7 or the pharmaceutical composition of item 9 for use in the prophylaxis or treatment of a disease, wherein the calcification of Bruch's membrane and/or adjacent tissues, including the retinal pigment epithelium and the choroid of an eye is a symptom of the disease.

11. The nanoparticle for use or the pharmaceutical composition of item 10, wherein the disease is selected from the group consisting of Pseudoxanthoma elasticum, Angioid Streaks, peau d'orange fundus, Beta-Thalassemia, Sickle Cell disease, Juvenile Paget's disease, Hyperphosphatemic familial tumoral calcinosis (HFTC), ENPP1 deficiency, Idiopathic Sclerochoroidal calcification, Choroidal calcification associated to chondrocalcinosis (pseudogout), Sclerochoroidal Calcification with optic nerve calcification in primary hyperparathyroidism or parathyroid adenoma, Genetic renal tubulopathies: like Bartter syndrome, and Gitelman syndrome, Aplasia cutis congenita and oculoectodermal syndrome, Cytomegalovirus Retinitis, Age-related macular degeneration, Refractile drusen (a feature of AMD), and optic nerve head drusen.

12. The nanoparticle for use of any one of items 1 to 7 or 10 or 11 or the pharmaceutical composition of any one of items 8 to 11, wherein the nanoparticle or the pharmaceutical composition is administered intravenously, intravitreally, subconjunctivally, subtenonly, retrobulbarly, posteriorly, juxtasclerally, suprachoroideally, subretinally, or topically as eye drops.

A better understanding of the present invention and of its advantages will be had from the following examples, offered for illustrative purposes only. The examples are not intended to limit the scope of the present invention in any way.

## EXAMPLES

## Material and Methods

### 1.1 Materials

Human serum albumin (HSA, purity > 96%), glutaraldehyde 50% (mA/) solution, and hydrocortisone were purchased from Sigma-Aldrich (Steinheim, Germany). Diethylenetriaminepentaacetic acid (DTPA) dianhydride was obtained from Thermo Fisher (Karlsruhe, Germany). The fluorescent dye PromoFluor-633P-NHS-ester (PF633P) was purchased from PromoCell GmbH (Heidelberg, Germany). The crosslinker NHS-PEG5000-Maleinimide (NHS-PEG$_{5000}$-Mal) was purchased from JenKem Technology (Plano, USA). The anti-elastin antibody (polyclonal antibody to elastin -purified) was obtained from OriGene Technologies GmbH (Herford, Germany). IgG from rabbit serum was purchased from Sigma-Aldrich (Steinheim, Germany). Immortalized human T-lymphocyte cells (Jurkat) were purchased from Deutsche Sammlung von Mikroorganismen und Zellkulturen GmbH (Braunschweig, Germany) and the immortalized human cerebral microvascular endothelial cell line (hCMEC/D3) was purchased from Merck KGaA (Darmstadt, Germany). Roswell Park Memorial Institute (RPMI)1640, gentamicin, non-essential amino acids, L-alanyl-L-glutamine, sodium pyruvate, penicillin and streptomycin solution, trypsin/EDTA solution, phosphate buffered saline (PBS), and fetal bovine serum (FBS) were obtained from Biochrom AG (Berlin, Germany). Thermo Fisher GmbH (Karlsruhe, Germany) provided o-cresolphthalein complexone grade as well as diethylenetriaminepentaacetic acid (DTPA).

### 1.2 Nanoparticle preparation

### 1.2.1 Preparation of HSA nanoparticles

The albumin-based nanoparticles (HSA-NP) were prepared by using a previously described desolvation technique (Marty *et al.,* 1978; Weber *et al.,* 2000). Briefly, the pH of an HSA solution (50 mg/ml, 1 ml) was adjusted to 7.3 with NaOH 2 N. The dropwise addition (1 ml/min) of 4 ml ethanol (96%, v/v) led to desolvation and thus formation of nanoparticles. The addition of 11.6 µl glutaraldehyde (8%, m/v) resulted in a theoretical crosslinking degree of 40%. After stirring with defined conditions (550 rpm, 22°C) overnight, the nanoparticles were purified by 2-fold centrifugation (21,000 × g, 16 min) and redispersed in a medium depending on further proceeding.

### 1.2.2 Preparation of PromoFluor 633P-labeled HSA-NP

HSA was labeled with the fluorescent dye PromoFluor-633P-NHS-ester (PF633P) prior to nanoparticle (NP) formation as described by Mesken *et al.* (Mesken *et al.,* 2017).1 mg PF633P was dissolved in 100 µl purified water. The solution was immediately added to 2 ml of an HSA solution (20 mg/ml) in bicarbonate buffer pH 8.3 and incubated for 1 h at 22°C, 600 rpm. Unbound PF633P was removed by purification using a dextran column (D-Salt™ Desalting Column, Thermo Scientific, Rockford, USA). After freeze drying of the labeled protein (pHSA), NP were prepared as described above by using a molar ratio of 1 pHSA to 5 unlabeled protein.

### 1.2.3 Covalent coupling of DTPA to the nanoparticle surface

A modified method of Hnatowich *et al.* (Michaelis *et al.*, 2004; Hnatowich *et al.*, 1983) was used to achieve a covalent binding of DTPA to HSA-NP. 9.08 mg DTPA dianhydride was added to 41.3 mg HSA-NP dispersed in NaHCO$_3$-buffer 0.05 M, pH 7.0 resulting in a molar excess of DTPA to HSA of 40:1. After stirring for 1 h (550 rpm, 22°C), unbound DTPA was removed by 3-fold centrifugation (20,000 × g, 14 min) and resuspension of the nanoparticles in purified water. After the final centrifugation, the DTPA bound HSA-NP (DTPA-(HSA-NP)), or if pHSA was used, DTPA-(pHSA-NP)) were redispersed in phosphate buffer pH 8.0 for antibody-conjugation.

### 1.2.4 Surface modification with antibodies (Synthesis route 1)

The conjugation of the antibody to the NP consisted of three steps. First, thiol groups were introduced onto the NP surface by a reaction of the HSA amine groups with 2-iminothiolane (Weber *et al.*, 2000a). A 50-fold molar excess of 2-iminothiolane over HSA was added to the NP dispersion in phosphate buffer pH 8.0. After incubation for 24 h at 22°C, 600 rpm in a thermomixer, the NP were purified twice by centrifugation (20,000 × g, 14 min) and redispersed in phosphate buffer pH 8.0. Meanwhile, either the anti-elastin antibody (el ab) or IgG were activated with the heterobifunctional crosslinker NHS-PEG$_{5000}$-Mal. The crosslinker was used in 50-fold molar excess. Immediately after dissolving in phosphate buffer pH 8.0, NHS-PEG$_{5000}$-Mal was added to 100 μg antibody followed by incubation for 1 h at 22°C, 600 rpm. Subsequent, the thiolated NP were combined with the PEG-antibody complex and after incubation overnight (22°C, 600 rpm), the particles were purified by one-time centrifugation (18,000 g, 14 min) and redispersion in purified water. The resulting particles are hereinafter referred to as summarized in Table 1.

The surface modified NP according to synthesis route 1 were used for the experiments on NP characterization (Figure 1 and 2) and cell interaction (Figure 3 - 7).

**Table 1** Abbreviations used for antibody-conjugated nanoparticles.

| Nanoparticle system | Matrix material | Chelating drug | Conjugated antibody |
|---|---|---|---|
| (HSA-NP)-el ab | HSA | - | Elastin ab |
| (pHSA-NP)-el ab | pHSA | | |
| (HSA-NP)-IgG | HSA | - | IgG |
| (pHSA-NP)-IgG | pHSA | | |
| DTPA-(HSA-NP)-el ab | HSA | DTPA | Elastin ab |
| DTPA-(pHSA-NP)-el ab | pHSA | | |
| DTPA-(HSA-NP)-IgG | HSA | DTPA | IgG |
| DTPA-(pHSA-NP)-IgG | pHSA | | |

### 1.2.5 Surface modification with antibodies (Synthesis route 2)

The second antibody conjugation method used was characterized by the reversal of the crosslinking partners. In this case, the NP surface was first modified with the heterobifunctional crosslinker NHS-PEG5000-Mal. For this purpose, a 15-fold molar excess of the crosslinker in relation to HSA was added to the NP suspension. After incubation for 1 h at 22 °C, 600 rpm in a thermomixer, the NP were purified by centrifugation (13,000 × g, 13 min) and redispersion in phosphate buffer pH 8.0.

In parallel, the antibody was thiolated with a 50-fold molar excess of 2-iminothiolane in phosphate buffer pH 8.0 for 2 h at 600 rpm and 22 °C.

For covalent coupling, the thiolated antibody was incubated with the NP suspension (20 μg modified antibody / mg NP) overnight at 600 rpm and 22 °C. The resulting NP were purified by centrifugation (10,000 × g, 10 min) and redispersion in purified water. The surface modified NP according to synthesis route 2 were used for the experiments on reversion of calcification in ttw/ttw mice (Figures 18 and 19).

### 1.2.6 Preparation of magnetite-HSA nanoparticles

HSA was dissolved in ultrapure water at a concentration of 50 mg/ml. The pH was then adjusted to 8.5 using 2 M NaOH. Subsequently, 167 μl of a magnetite suspension (aqueous; 3% m/V) was added to 1 ml of the HSA solution. The pure mass of magnetite corresponded to 10% of the total mass of HSA. The magnetite-HSA solution was incubated for 1 h (20° C; 700 rpm).

The albumin-based nanoparticles (HSA-NP) were prepared by using a previously described desolvation technique (Weber *et al.,* 2000). Briefly, the pH of an HSA solution (50 mg/ml, 1 ml) was adjusted to 7.3 with NaOH 2 N. The dropwise addition (1 ml/min) of 4 ml ethanol (96%, v/v) led to desolvation and thus formation of nanoparticles. The addition of 11.6 $\mu$l glutaraldehyde (8%, m/v) resulted in a theoretical crosslinking degree of 40%. After stirring with defined conditions (550 rpm, 22°C) overnight, the nanoparticles were purified by 2-fold centrifugation (21,000 $\times$ g, 16 min) and redispersed in phosphate buffer pH 8.0.

The magnetite-HSA nanoparticles were used for the accumulation experiments in Bruch's membrane of ttw/ttw mice (Figure 17).

### 1.3 Physicochemical characterization of nanoparticles

### 1.3.1 Photon correlation spectroscopy (PCS)

The average hydrodynamic diameter and the polydispersity index (Pdl) were determined by PCS measurement. After aqueous dilution, the NP suspensions were analyzed at a temperature of 22°C and a backscattering angle of 173°. The zeta potential - a dimension for the nanoparticle surface charge - was measured by laser Doppler microelectrophoresis using the zeta potential mode. Both experiments were conducted using a Malvern Zetasizer Nano ZS system (Malvern Instruments Ltd., Malvern, UK).

### 1.3.2 Scanning electron microscopy images

Samples for the scanning electron microcopy images were prepared by applying 3 $\mu$l of a NP suspension (0.25 mg NP/ml) onto a polycarbonate membrane filter (pore width 0.1 $\mu$m). After drying in a desiccator overnight, the filter was sputtered with a thin gold layer. The images were taken with a CamScan CS4 scanning electron microscope (operating distance 10 mm, accelerating voltage 10 kV, Cambridge Scanning Company, Cambridge, UK).

### 1.3.3 Quantification of bound DTPA

The amount of bound DTPA was determined indirectly using a modified high-performance liquid chromatography (HPLC) method previously described by Yamaguchi *et al.* (Yamaguchi *et al.,* 1983). The supernatants resulting from purification after the coupling reaction were collected and a molar amount of iron(III) chloride equivalent to the maximum contained DTPA was added. Samples were analyzed using a HPLC-DAD system (Agilent Technologies 1200 series) combined with a reversed phase column (Gemini® RP 18; 250 $\times$ 4.6 mm, particle diameter 5 $\mu$m (Phenomenex, Aschaffenburg, Germany), column temperature 30°C). A mobile phase consisting of phosphate buffer 0.02 M, pH 2.5, 0.175 mM tetrabutylammonium bromide and 5% acetonitrile was utilized for isocratic elution at a flow rate of 1.0 ml/min. Quantification was carried out using a calibration curve in a range of 0.1 - 1 mg DTPA/ml with a diode array detector (DAD) set to a wavelength of 257 nm for detection of the iron-DTPA-complex.

### 1.3.4 Verification of covalent binding of DTPA

The covalent binding of DTPA to the NP was confirmed by two methods.

First, the adsorption of DTPA to the NP surface was analyzed. Samples were prepared as described for the covalent DTPA coupling with the difference that DTPA dianhydride was not added to the NP suspension as dry powder but stirred in NaHCO$_3$-buffer 0.05 M, pH 7.0 for at least 1 h beforehand resulting in hydrolysis of the anhydride function. Afterwards, purification was performed as described above and the amount of DTPA adsorbed to the NP was determined using the specified HPLC method.

The second approach employed an *in vitro* release study to prove the persistence of the covalent linkage. NP dispersed in Dulbecco's Modified Eagle Medium (DMEM) at a concentration of 1 mg/ml were incubated using a thermomixer at 37°C, 600 rpm for defined time periods (30 min, 1 h, 2 h, 4 h, 6 h, 24 h). After the specified times, samples were centrifuged (20,000 $\times$ g, 15 min) to separate the NP. The supernatant was analyzed for free DTPA via HPLC-DAD as mentioned above.

### 1.3.5 Colloidal stability

The colloidal stability against the influence of electrolytes and pH was tested by performing titration experiments using the Malvern Zetasizer Nano ZS system in combination with a Multi-Purpose Titrator MPT-2 (Malvern Instruments Ltd., Malvern, UK). An aliquot of the NP suspension containing 5 mg NP was dispersed in purified water at a concentration of 0.38 mg/ml. For evaluating the stability against electrolytes, the titration was implemented using 0.6 M NaCl as titrant covering a range from 0 to 200 mM in 25 mM steps. The dispenser was set automatically and recirculation between measurements was activated for all measurements. For each electrolyte concentration the hydrodynamic diameter, Pdl, and zeta potential were measured. Titrations from pH 2 - 12 (increment: 0.5 pH intervals) were conducted using NaOH 0.05 N and HCl 0.05 N as titrants. The same parameters were determined as described for the electrolyte titration.

### 1.3.6 *In vitro* chelating ability

To evaluate the *in vitro* chelating ability of the prepared NP formulations, a method by Sarkar and Chauhan (*Sarkar et al.,* 1967) was adapted. Briefly, an aliquot (1 ml) of a calcium chloride solution (10 μg/ml) was diluted to 1.375 ml with purified water, followed by the addition of 0.625 ml of an ammonium buffer pH 10.5 (0.24 g $NH_4Cl$ in 100 ml $NH_4OH$ 5%). 1 mg of NP was added and the volume filled up to 2.25 ml using purified water. After incubation for 5 min, NP were removed by centrifugation (20,000 $\times$ g, 15 min). An aliquot (0.25 ml) of o-cresolphthalein complexone (OCPC) solution 0.1% was added. Samples were analyzed using a Synergy MX multi-well spectrophotometer (BioTek Instruments GmbH, Bad Friedrichshall, Germany) set at a wavelength of 555 nm. A calibration curve of calcium ranging from 0 - 10 μg/ml calcium was prepared by following the steps described above but without NP addition.

### 1.3.7 Extent of antibody conjugation to the nanoparticles

The supernatants obtained through purification of the antibody-modified NP were analyzed with a GPC-VWD system to indirectly determine the extent of the antibody binding to the NP. An aliquot (20 μl) of the sample was injected onto a PSS Proteema column (PSS Proteema 300 Å, 5 pm, 300 $\times$ 8 mm, Polymer Standards Service, Mainz, Germany) and eluted isocratically within 20 min at a flow rate of 1 ml/min, column temperature of 30°C. Phosphate buffer pH 6.6 was used as mobile phase. Detection wavelength was set at 280 nm. The column's separation capacity was tested by separation of the protein standard (Bio-Rad Laboratories GmbH, München, Germany) prior to each sequence.

The number of antibodies per one NP was calculated by first calculating the volume of one HSA-NP ($V_{NP}$) using the following equation (1):

$$V_{NP} = \frac{4}{3}\pi \times \left(\frac{d_h}{2}\right)^3, (1)$$

where $d_h$ represents the hydrodynamic diameter of HSA-NP.

Next, the mass of one HSA-NP ($m_{NP}$) was calculated using the equation (2) below:

$$m_{NP} = V_{NP} \times \rho_{NP}, (2)$$

where $\rho_{NP}$ represents the density of HSA-NP which is 1.31 g/cm$^3$ (Weber *et al.,* 2000a). By dividing the amount of the used NP for the antibody conjugation ($m_{NP\ ab\text{-}conj.}$) by $m_{NP}$, the total number of NP ($Z_{NP}$) was calculated (3):

$$Z_{NP} = \frac{m_{NP\ ab-conj.}}{m_{NP}}, (3)$$

The number of antibody molecules ($Z_{ab}$) was calculated by dividing the mass of used antibody ($m_{ab}$) by the molar mass of the antibody ($M_{ab}$), followed by multiplication with the Avogadro constant ($N_A$) (4):

$$Z_{ab} = \frac{m_{ab}}{M_{ab}} \times N_A, (4)$$

The number of antibody molecules per one NP (ab/NP) was calculated using the following equation (5):

$$\frac{ab}{NP} = \frac{Z_{ab}}{Z_{NP}}, (5)$$

### 1.4. Cell culture experiments

### 1.4.1 Cell culture routines

hCMEC/D3 cells were cultivated using RPMI 1640 supplemented with 1% (v/v) non-essential amino acids, 1% (v/v) L-alanyl-L-glutamine (200 mM), 1% (v/v) gentamicin, 1% (v/v) sodium pyruvate (100 mM), 550 nM hydrocortisone, and 20% (v/v) FBS. Jurkat cells were cultivated using RPMI 1640 supplemented with 1% (v/v) of a mixture of penicillin (100 U/ml) and streptomycin (100 $\mu$g/ml), 1% (v/v) L-alanyl-L-glutamine (200 mM), 1% (v/v) gentamicin, and 10% (v/v) FBS. Both cell lines were stored in T75 cell culture flasks in an incubator at a temperature of 37°C and 100% humidity at 5% $CO_2$ atmosphere. The cells were split twice a week at a ratio of approximately 1:3. After thawing, all experiments were conducted within a maximum of 20 passages.

Human macrophages were isolated from buffy coats from peripheral blood of healthy volunteers obtained from the Red Cross by Ficoll gradient centrifugation subsequently followed by the isolation of CD14 positive cells by Magnetic Associated Cell Sorting (MACS, Miltenyi Biotec GmbH, Bergisch Gladbach, Germany). The purity of at least 95% of positive cells was determined by flow cytometry in an Agilent 2100 Bioanalyzer (Agilent Technologies Deutschland GmbH, Waldbronn, Germany). After isolation, cells were cultured in McCoy's 5A medium supplemented with 15% FBS, 1% L-glutamine, and 2% non-essential amino acids. The experiments were performed immediately after the isolation procedure at 37°C and 7% $CO_2$. Primary human umbilical vein endothelial cells (HUVECs) were obtained from PromoCell GmbH (Heidelberg, Germany). Briefly, the cells where cultured in Endothelial Cell Growth Medium 2 (ready-to-use) (PromoCell GmbH, Heidelberg, Germany) to confluency, subsequently followed by cytotoxicity experiments.

### 1.4.2 Cytotoxicity studies

To evaluate the cytotoxicity of the NP formulations a WST-1 assay (Roche Diagnostics, Mannheim, Germany) was conducted on Jurkat and hCMEC/D3 cell lines. Human macrophages and HUVECs were tested applying an XTT assay (Sigma-Aldrich Chemie GmbH, Munich, Germany). After seeding Jurkat and hCMEC/D3 cells in a 96 well plate at a density of $2 \times 10^4$ cells/well (hCMEC/D3 cells suspended in 100 $\mu$L, Jurkat cells suspended in 50 $\mu$L), they were cultured for 48 h with serum containing medium. The NP influence on the cell viability was tested at concentrations ranging from 0.5 $\mu$g/ml to 250 $\mu$g/ml. Since Jurkat cells are suspension cells while hCMEC/D3 adhere to the well plate, the procedure for the two cell lines differed. Prior to the addition of the NP to hCMEC/D3 cells, the medium was removed. 100 $\mu$l of the NP suspension in serum free medium was added. After incubating for 24 h, the cells were washed once with serum free medium and 100 $\mu$l fresh serum free medium was added again. Following a blank measurement at 460 nm with a Synergy MX multi-well spectrophotometer, 10 $\mu$l of WST-1 reagent was pipetted into each well. After 30 min, the absorbance was measured again. To evaluate the viability, a negative and a positive control were conducted simultaneously to the nanoparticle incubation (100 $\mu$l of DMSO (0% viability) and 100 $\mu$L of serum free medium (100% viability)). The blank measurement was subtracted and the absorbances were correlated to the controls. For Jurkat cells, NP dilutions in serum free medium were twice as high as for hCMEC/D3 cells and 50 $\mu$l was added to the already present medium from seeding resulting in a 1:1 dilution to the final concentration. For the positive and negative control, 50 $\mu$l of DMSO and 50 $\mu$l of serum free medium was added, respectively. After 24 h of incubation, the blank measurement was performed without any washing steps. The remaining steps were equal to the ones described for the hCMEC/D3 cells.

The XTT assay was applied according to the manufacturer's protocol. Briefly, $2 \times 10^4$ cells, suspended in 100 $\mu$l serum free medium, were seeded in a 96 well plate. In contrast to Jurkat and hCMEC/D3 cells, the viability of macrophages and HUVECs was investigated in the presence of only the highest concentration of 250 $\mu$g NP/ml. The cells were incubated for 24 h with the respective nanoparticles, followed by a washing step with serum free medium. After adding 100 $\mu$l fresh serum free medium, a blank measurement was conducted at 450 - 500 nm. XTT reagent was added in aliquots of 50 $\mu$l/well and incubated for 18 h at 37°C and 7% $CO_2$. The absorbance was measured and, after subtraction of the blank measurement, correlated to a negative control (cells in pure serum free medium).

### 1.4.3 Cellular interaction

To determine the cellular interaction of the NP formulations, three methods were employed. The first approach was conducted using an IncuCyte® S3 Live-Cell Analysis Imaging System (Essen Bioscience, Inc., Michigan, USA). The second experiment was flow cytometry analysis. The obtained results were supported by visualization via fluorescence microscopy. All experiments were conducted using PF633P-labeled and therefore fluorescent NP.

### 1.4.3.1 Live-cell imaging

For analysis of NP interaction with cells, an IncuCyte® S3 Live-Cell Analysis Imaging System was used. hCMEC/D3 cells were seeded at a density of $2 \times 10^5$ cells/well in a 24 well plate. After cultivating for 48 h, the medium was exchanged with medium containing NP in a concentration of 0.02 mg/ml. Jurkat cells (50 $\mu$l) were seeded in an agarose-coated 96 well plate at a density of $1 \times 10^4$ cells/well. An aliquot (50 $\mu$l) of a 0.04 mg/ml NP suspension was added resulting in a dilution to 0.02 mg NP/ml. The well plates were scanned every hour over a total time of 24 h whereby nine images per well were taken for the 24 well plate and 4 images for the 96 well plate at each time point. Fluorescent-labeled nanoparticles were detected by the image channel red (excitation: 565 - 605 nm / emission: 625 - 705 nm). The obtained red object areas of the samples minus the background of control wells containing medium without nanoparticles were set in relation.

### 1.4.3.2 Fluorescence-activated cell sorting (FACS)

To investigate the cellular interaction of the nanoparticles in more detail, FACS analysis was performed. hCMEC/D3 cells were seeded in a 24 well plate at a density of $2 \times 10^5$ cells/well and cultivated for 48 h. 24 h before the measurement, the medium of three wells was exchanged with medium supplemented with 0.02 mg NP/ml for the first time point. For the remaining time points, the process was repeated 6 h, 4 h, 2 h, and 1 h prior measuring. After incubation, the medium was removed and cells were washed once with 300 $\mu$l trypsin/EDTA (0.05%/0.02%) solution. For enzymatic detachment of the cells another 300 $\mu$l trypsin/EDTA solution was added before placing the well plate in the incubator (37°C, 5% $CO_2$) for 7 min. 700 $\mu$L of medium was added to stop the enzymatic digestion and thereafter, samples were transferred into FACS tubes (Sarstedt, Nuembrecht, Germany) and analyzed using a FACSCalibur™ system and CellQuest™ Pro software, red laser 640 nm, filter 670LP, detector FL4 (Becton Dickinson Heidelberg, Germany). A minimum of 10,000 cells per sample was measured. The obtained mean fluorescence intensities were standardized to the fluorescence intensity of untreated control cells. For Jurkat cells, the experiment started directly after transferring 250 $\mu$l cell suspension (containing $1 \times 10^5$ cells) in 2 ml micro tubes. The NP suspension was concentrated twice as high as required since 250 $\mu$l were added to the already present medium resulting in a 1:1 dilution to 0.02 mg NP/ml. The addition followed the same time protocol as described for hCMEC/D3 cells. Afterwards, the NP cell suspensions were directly transferred into FACS tubes. Analysis was carried out as described above.

### 1.4.3.3 Visualization of cellular interaction

For fluorescence microscopy, hCMEC/D3 cells were seeded with a density of 2.5 x $10^4$/chamber on Millicell® EZ slides (Merck, Darmstadt, Germany). After a 24 h growing period, 0.625 $\mu$g NP (2.5 $\mu$g NP/ml) were incubated for 24 h. After washing the cells with PBS++, they were incubated for 10 min with wheat germ agglutinin (WGA) Alexa Fluor® 350 solution (10 $\mu$g/ml) to stain the cell membranes. Following another washing step with PBS++, cells were fixed with paraformaldehyde 4% (m/v) for 15 min at room temperature. A final washing step was followed by covering the cells with Vectashield® mounting medium with DAPI (Vector Laboratories Inc., Burlingame, USA). Since Jurkat cells are non-adherent cells, slides were coated with poly-L-lysine (PLL). Incubation with NP was carried out in 2 ml micro tubes. Therefore, 5 x $10^4$ cells were incubated with 1.25 $\mu$g NP (5 $\mu$g NP/ml) for 24 h. After centrifugation (1,000 $\times$ g, 5 min, 22°C), cells were resuspended in PBS++. After incubation with wheat germ agglutinin (WGA) Alexa Fluor® 350 solution (10 $\mu$g/ml) for 10 min, 20 $\mu$l of the cell suspension was pipetted onto the PLL-coated slides and left to adhere for 5 min. After a washing step with PBS++, the remaining procedure for fixation and mounting was performed as described for hCMEC/D3. Samples were analyzed using an IX81 fluorescence microscope (Olympus, Hamburg, Germany) with filter systems including excitation at 360 - 370 nm, dichroic mirror at 400 nm, emission at 426 - 446 nm for DAPI and Alexa Fluor® 350 and excitation at 575 - 630 nm, dichroic mirror at 636 nm, emission at 645 - 695 nm for PF633P. All pictures were taken as multi-layer image stacks with a minimum of 12 images. The stacks were processed by deconvolution ("Wiener filter") using cellSens Dimension Software version 1.8.1 (Olympus, Hamburg, Germany) to reduce out of focus fluorescence.

### 1.5 *In vivo* experiments

### 1.5.1 Animals and diet, dilution and application of NP, procedure of tail vein injection

The ttw/ttw mice used in this study have been described previously (Hosoda *et al.,* 1981; Okawa *et al.,* 1998). Ttw/ttw mice were bred onto a C57BL/6J background for more than ten generations, and ttw/ttw and wildtype (WT) littermate control (male and female) animals were generated through heterozygous mating. The study was approved by the local committee for animal studies (Reg. No.:81-02.04.2018.A233) and was performed according to the guidelines from Directive 2010/63/EU of the European Parliament on the protection of animals used for scientific purposes. Mice were genotyped after weaning and ttw/ttw mice were fed a high phosphate ("acceleration") diet (Altromin C1031 mod. #103153 with 0.4% calcium, 0.85% phosphorous and 0.04% magnesium) starting with 5 weeks of age.

Mice receiving nanoparticle treatment were injected i.v. twice a week, starting from 10 weeks of age (4 injections in total) into the lateral tail vein. For injection, DTPA-(HSA-NP)-ElastinAb were used. NP were injected in a maximum volume of 100 $\mu$l, in a concentration of 20 $\mu$g/g body weight, diluted in 0.9% NaCl. For NP injection, mice were weighed and placed in an animal restraint device. The veins of the mice were vasodilated prior to intravenous injection by prewarming of the tail in warm water (around 30°C) for 1-2 min, followed by disinfection of the tail. The needle was inserted into the distal portion of the middle 1/3 of the tail and the substance was injected. After injecting, the needle was left in tail for 2-3 seconds to allow the injected substance to clear the vein, then the needle was removed and immediately pressure was applied to the puncture site to prevent unnecessary blood loss. Once bleeding had stopped, the animal was removed from the restrainer. Mice were observed 5-10 min to ensure hemostasis (bleeding has stopped) and normal behavior of the animal. All animals were sacrificed with 9 or 12 weeks of age by transcardial perfusion with 0.9% NaCl under deep anesthesia (5% isoflurane).

Additionally, some ttw/ttw mice received HSA-based anti-elastin conjugated NP coupled to magnetite (see 1.2.6) to visualize intra-ocular NP localization after systemic injection of the NP-solution. These mice were sacrificed 4 or 48 hours after injection and eyes were sectioned and prepared for electron microscopy.

### 1.5.2 Histology of mouse eyes

#### 1.5.2.1 Sectioning, preparation of samples and mounting

For histology, organs were fixed in 4% PFA for 24 hours, embedded in Cryomatrix™ (Thermo Fisher Scientific, Schwerte, Germany) and stored at -80°C until further processing. Cryo-embedded organs were cut into serial sections of 7 - 25 $\mu$m at the cryotome and transferred onto object slides. Slides were stored at -20 °C until further treatment.

For flat mount staining of the eyes, eyes were enucleated and connective tissue was removed. Then the cornea, iris, lens and retina were gently removed. The eyecup was cut in each quarter to allow it to lie flat. The eye was incubated in 0.1% trypsin at room temperature for 15 min, followed by gentle brushing of the retinal pigment epithelium. Flat mounts were then fixed for 1 hour and stored at 4°C in PBS until further processing.

For cross sections of the flat mount specimens, samples were uncovered from slides in PBS overnight followed by embedding in Cryomatrix™. Cryo-embedded flat mounts were then cut into serial sections of 10 $\mu$m at the cryotome and transferred onto object slides. Slides were stored at -20 °C until further treatment.

#### 1.5.2.2 Alizarin red staining

Slides were washed five minutes in PBS, fixed in 95% ethanol for 10 min at room temperature and then air dried. Samples were incubated five minutes with 2% alizarin red S pH 4.2, rinsed using aqua dest. and dehydrated (2 min in 70% EtOH, 5 min in 96% EtOH, 5 min in 99% EtOH, 5 min in 99% EtOH, 2 × 5 min in Roti®Histol (Carl Roth, Karlsruhe, Germany)). Slides were covered with ROTI®Histokitt (Carl Roth, Karlsruhe, Germany) and staining of red calcium mineral deposits was recorded using the Nikon eclipse ci microscope with Nikon NIS-Elements - Imaging Software (Nikon Europe BV Amsterdam, Netherlands).

#### 1.5.2.3 Osteosense staining

Slides were washed in 1x PBS for five minutes. Then, slides were incubated with Osteosense 680EX (0.16 nmol/ml; NEV10020EX, Perkin Elmer, Waltham, US), a fluorescence labeled hydroxyapatite antibody, at room temperature for 20 minutes. Afterwards the slides were washed in PBS twice for five minutes followed by incubation with DAPI (1:1000 in PBS; 4',6-diamidino-2-phenylindole, D1306, Thermo Fisher Scientific, Schwerte, Germany) for nuclei staining for 15 minutes in the dark. Slides were washed three times for five minutes in PBS and covered with fluorescent mounting medium. Analysis of the staining was done using a Zeiss LSM 880 with Zen software (Carl Zeiss Microscopy GmbH, Jena, Germany).

Flat mounts were gently flattened on a slide and stained using the same protocol.

### 1.5.3 Microscopy

#### 1.5.3.1 Light Microscopy

Aldehyde-fixed, dehydrated, and epoxy-infiltrated retinas were sectioned to a thickness of 1 $\mu$m using ultra-microtomy and block trimmed to two regions of retina: in the mid-retina in the posterior of the eye and in a region of retina near the ora serrata. Sections were stained with 3% silver nitrate (von Kossa stain) for ~ 30 min and 1% toluidine blue solution for ~ 15 seconds. Bright-field images were acquired on a Leica DMi8 inverted microscope.

#### 1.5.3.2 X-ray microscopy

WT and ttw/ttw eyes were fixed for 2 h in a mixture of 5% glutaraldehyde and 4% paraformaldehyde in 0.1 M sodium cacodylate buffer, before being serially dehydrated to 100% ethanol. Whole eyes were then critical point dried and mounted to wooden splints by applying UV-curable dental epoxy to the optic nerve. Specimens were scanned using a Zeiss XRadia Versa 520 X-ray microscope. Whole eyes were scanned in air using a voltage of 65 kV, a power of 5 W, a 1 s exposure time, and a 4X objective across 3001 projections to achieve a voxel size of 3.40 $\mu$m. Inset volumes of calcification in the ttw/ttw eye were scanned at a voltage of 65 kV, a power of 5 W, a 7.5 s exposure time, and a 20X objective across 3001 projections to achieve a voxel size of 0.56 $\mu$m. Reconstruction of ttw/ttw and WT datasets was conducted in Dragonfly software version 2022.2 (Comet Technologies Inc.). Mineral in the ttw/ttw eyes were segmented using grayscale thresholding.

### 1.5.3.3 Scanning electron microscopy

A ttw/ttw eye was serially dehydrated in acetone and gradually infiltrated with epoxy (Embed812) for electron microscopy. After curing, the block was trimmed to an appropriate region in the posterior segment of the eye and sputter coated with a 4 nm layer of platinum to mitigate charging. Imaging of sub- retinal pigment epithelium (RPE) deposits was conducted in a FEI Quanta 450 scanning electron microscope at an accelerating voltage of 20 kV, with energy dispersive X-ray spectroscopy (EDX) conducted at 10 kV to localize calcium and phosphorus within the deposit.

### 1.5.3.4 Transmission Electron Microscopy of mouse eyes

Aldehyde-fixed, dehydrated, and epoxy-embedded eyes were block trimmed to areas near the ora serrata and in the mid-retina. Sections for transmission electron microscopy (TEM) were cut to a thickness of 100 nm using ultramicrotomy and mounted on copper grids before staining with uranyl acetate and lead citrate to add imaging contrast to the organic phases. Bright-field TEM and high-angle annular dark field scanning transmission electron microscopy (HAADF-STEM) were conducted on a Thermo Scientific Talos F200X microscope at an accelerating voltage of 200 kV.

### 1.5.4 X-ray diffraction

The epoxy embedded ttw/ttw eye from scanning electron microscopy was gently polished to remove the platinum coating and X-ray diffraction spectra were acquired using a Bruker D8 Discover instrument with a copper X-ray source, area detector, and 500 $\mu$m probe size. Four frames were acquired by positioning the beam on the drusen using a frame width of 23° and a frame acquisition time of 5 min. The resulting 2D diffraction pattern was integrated to form a 1D spectrum using DIFFRAC.EVA software.

### 1.5.5 Transmission Electron Microscopy of mouse eyes for visualization of magnetite coupled nanoparticles

Eyes were isolated and immediately fixed in 2% (v/v) formaldehyde and 2.5% (v/v) glutaraldehyde in 100 mM cacodylate buffer, pH 7.4, at 4°C overnight. After washing in PBS, the eyes were post fixed in 0.5% (v/v) osmium tetroxide and 1% (w/v) potassium hexacyanoferrate (III) in 0.1 M cacodylate buffer for 2 h at 4°C followed by washing with distilled water. After dehydration in an ascending ethanol series from 30% to 100% ethanol, specimens were incubated two times in propylenoxide each for 15 min and embedded in Epon. Ultrathin sections were cut with an ultramicrotome, collected on copper grids, and negatively stained with 2% uranyl acetate for 10 min. Electron micrographs were taken at 60 kV with a Phillips EM-410 electron microscope using a Veleta camera system in combination with the Radius software system (emsis, Münster, Germany).

### 1.6 Calcification quantification

For calcium quantification, organs were dried at 60°C for two days. To solubilize phosphocalcific deposits, the organs were weighed and incubated overnight in 150 - 600 $\mu$l 0.6 N HCl, depending on the size of the organ. The organs were then sonicated for 10 seconds and centrifuged for 10 minutes with 13,000 rpm. 1% quinolinol solution (0.1 g quinolinol in 10 ml pure ethanol) and 0.1% o-cresolphthalein solution (0.01 g o-cresolphthalein in 7.2 ml aqua dest. and 2.8 ml ammonium chloride buffer [0.48 g NH$_4$Cl in 200 ml NH$_4$OH 5%]) were prepared. Various concentrations of CaCl$_2$*2H$_2$O in 0.6 N HCl were used as standard. 50 $\mu$l of each standard or sample were incubated with 110 $\mu$l ammonium chloride buffer and 20 $\mu$l of the 1% quinolinol solution for five minutes. At last, 20 $\mu$l of o-cresolphthalein were added and incubated for another five minutes. Calcium quantification was performed in duplicates. Calcium content was determined colorimetrically at a wavelength of 570 nm and normalized on dry weight.

### 1.7 Statistical methods

All experiments were performed in triplicate. The obtained results are presented as average value with standard deviation. To compare independent groups, a one-way ANOVA using the Holm-Sidak post-test was performed with the software SigmaPlot 12.5 (Systat Software GmbH Erkrath, Germany). The significance levels are declared as * for $p < 0.05$, ** for $p < 0.01$, and*** for $p < 0.001$.

### Experimental examples

**Example 1** Preparation and characterization of nanoparticles

For the preparation of HSA nanoparticles according to point 1.2.4 of the Materials and Methods section, a desolvation method was utilized. HSA-NP with a crosslinking degree of 40% were obtained. These NP served as standard for the conducted experiments. The coupling reaction of DTPA dianhydride to the lysine residues of HSA lead to DTPA-(HSA-NP). The NP were conjugated to either the specific anti-elastin antibody or the unspecific IgG via a PEG-crosslinker. The results of the detailed physicochemical characterization of the prepared nanoparticle formulations, including the magnetite containing nanoparticles, are shown in **Table 2** (below). The particle systems showed an average hydrodynamic diameter between 149 and 178 nm. A Pdl below 0.1 reflected a monodisperse size distribution for all samples. By means of SEM images, the PCS data were verified (**Figure 1**). Additionally, the spherical morphology of the nanoparticles was proven. HSA-NP exhibited a zeta potential of $-22.6 \pm 5.3$ mV. The coupling of DTPA to the NP's surface led to a significant increase of the zeta potential of about 23 mV to $-47.9 \pm 7.0$ mV ($p \leq 0.001$). The antibody conjugated formulations were obtained with a zeta potential greater than -30 mV. Regarding the magnetite containing nanoparticles, the hydrodynamic diameter were 286 nm and 302 nm with a Pdl of 0.153 and 0.123. The zeta potential of the magnetite containing nanoparticles were -32.5 mV and -35.8 mV.

The amount of bound DTPA was quantified by HPLC analysis of the collected supernatants during preparation (**Table 2**). PromoFluor-labeled formulations had a significant lower DTPA binding of $4.6 \pm 0.1$ mol DTPA/mol HSA ($27.8 \pm 0.6$ μg DTPA/mg HSA) compared to $7.2 \pm 1.1$ mol DTPA/mol HSA ($43.6 \pm 6.7$ μg DTPA/mg HSA) for the unlabeled formulations ($p \leq 0.05$).

The covalent binding of DTPA to the nanoparticles was verified by two experiments. First, an adsorption study of DTPA to the NP's surface was conducted revealing that no DTPA was adsorbed to the NP surface. The whole DTPA amount used for the adsorption experiments was quantitatively recovered in the NP supernatant (data not shown). The second proof was given by a release study performed with the DTPA-modified NP. HPLC analysis of the supernatant after 24 h of nanoparticle incubation in serum free medium showed no DTPA peak in the chromatogram (data not shown). Consequently, no DTPA was released from the nanoparticles.

The extent of the NP surface functionalization with antibodies was evaluated. GPC analysis revealed that the antibody was quantitatively conjugated to the nanoparticle surface. Assuming an even distribution, 108 antibody molecules were bound per nanoparticle.

**Table 2** Physicochemical characteristics of prepared nanoparticles (mean $\pm$ SD; $n \geq 3$).

| Nanoparticle system | Hydrodynamic diameter [nm] | Pdl | Zeta potential [mV] | Drug load [mol DTPA/mol HSA] |
|---|---|---|---|---|
| HSA-NP/pHSA-NP | $158.1 \pm 20.0$ | $0.05 \pm 0.03$ | $-22.6 \pm 5.3$ | -- |
| DTPA-(HSA-NP)/ DTPA-(pHSA-NP) | $166.9 \pm 16.8$ | $0.08 \pm 0.06$ | $-47.9 \pm 7.0$ | $7.2 \pm 1.1/4.6 \pm 0.1$ |
| (HSA-NP)-el ab/ (pHSA-NP)-el ab | $168.8 \pm 22.1$ | $0.10 \pm 0.09$ | $-42.3 \pm 10.1$ | -- |
| (HSA-NP)-IgG/ (pHSA-NP)-IgG | $149.3^a$ | $0.04^a$ | $-31.0^a$ | -- |
| Magnetite(10%)-HSA-el ab-NP | $302.5^a$ | $0.153^a$ | $-35.8^a$ | -- |
| Magnetite(10%)-HSA-IgG-NP | $286.7^a$ | $0.123^a$ | $-32.5^a$ | -- |
| DTPA-(HSA-NP)-el ab/ DTPA-(pHSA-NP)-el ab | $155.3 \pm 25.5^b$ | $0.04 \pm 0.02^b$ | $-49.0 \pm 13.6^b$ | $7.2 \pm 1.1/4.6 \pm 0.1$ |

(continued)

| Nanoparticle system | Hydrodynamic diameter [nm] | PdI | Zeta potential [mV] | Drug load [mol DTPA/mol HSA] |
|---|---|---|---|---|
| DTPA-(HSA-NP)-IgG/ DTPA-(pHSA-NP)-IgG | $178.4 \pm 12.7^b$ | $0.08 \pm 0.05^b$ | $-40.5 \pm 1.5^b$ | $7.2 \pm 1.1/4.6 \pm 0.1$ |
| Exception: $^a$ n = 1, $^b$ n = 2 | | | | |

**Example 2** Colloidal stability

To evaluate the particle systems' stability, electrolyte titrations and pH titrations were conducted. As seen in **Figure 2A,** the nanoparticles were stable over a sodium chloride concentration range up to 200 mmol/l. **Figure 2B** shows the pH titration ranging from pH 2 to 11. The isoelectric points (IEP), represented by vertical lines, of HSA-NP and DTPA-(HSA-NP) were detected at $5.52 \pm 0.07$ and $5.28 \pm 0.11$, respectively. Between pH 4.3 and 5.5, the hydrodynamic diameter of DTPA-(HSA-NP) increased reaching a maximum of $4,142 \pm 696$ nm which is significantly higher than the diameter below pH 4.3 or above pH 5.5 ($p \leq 0.001$).

**Example 3** *In vitro* chelating ability

For a successful application in the dissolution of Bruch's membrane calcifications, DTPA-(HSA-NP) needed to exhibit chelating properties. Therefore, the chelating ability of the bound DTPA was evaluated. After incubating the NP in a calcium containing solution, they were removed by centrifugation and, after the addition of OCPC, the remaining calcium was quantified spectrophotometrically. As a result, 1 mg DTPA-(HSA-NP) chelated more than twice as much calcium compared to HSA-NP ($2.69 \pm 0.28$ $\mu$g calcium/mg NP compared to $1.31 \pm 0.13$ $\mu$g calcium/mg NP) (**Table 3**).

**Table 3** Chelating ability of HSA-NP and DTPA-(HSA-NP) (mean $\pm$ SD, n = 3; *** for $p \leq 0.001$).

| Nanoparticle formulation | Chelated calcium [$\mu$g/mg NP] |
|---|---|
| HSA-NP | $1.31 \pm 0.13$ |
| DTPA-(HSA-NP) | $2.69 \pm 0.28$*** |

**Example 4** Cell culture experiments

The nanoparticles used in Example 4 were prepared according to point 1.2.4 of the Materials and Methods section.

**Example 4a** Cytotoxicity

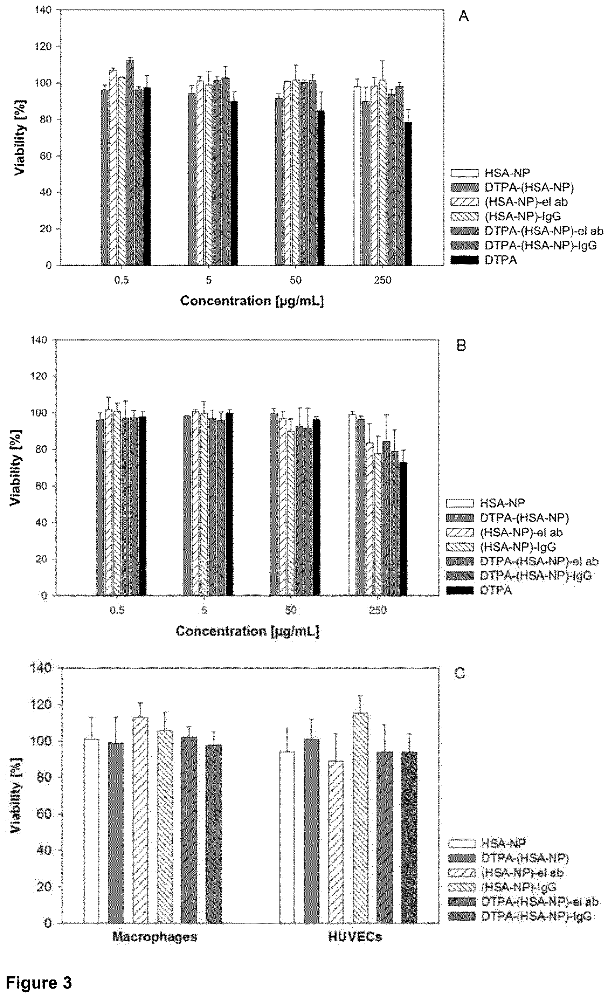

Since the NP were established to be applied into the human body, their biological safety is crucial. Thus, WST-1 and XTT assays were conducted to test the NP cytotoxicity. For Jurkat cells and hCMEC/D3, NP concentrations ranging from 0.5 to 250 $\mu$g/ml were tested. Pure DTPA was tested over the same concentration range. For primary macrophages and HUVECs, a NP concentration of 250 $\mu$g/ml was investigated. The results of the cytotoxicity studies are shown in **Figure 3A** and **Figure 3B** for hCMEC/D3 cells and Jurkat cells, respectively. The tested nanoparticulate formulations did not feature cytotoxic effects on hCMEC/D3 cells. Incubation with DTPA-(HSA-NP) resulted in the lowest viability with $89.9 \pm 7.9$% viable cells for 250 $\mu$g NP/ml related to incubation with pure medium. DTPA-(HSA-NP)-el ab and DTPA-(HSA-NP)-IgG impacted the cell viability comparable to the respective formulation without DTPA (around 98% cell viability). Pure DTPA lowered cell viability to $78.4 \pm 6.9$% at its highest concentration.

Jurkat cells exhibited a different sensitivity towards the formulations. Whereas DTPA-(HSA-NP) slightly reduced the number of viable cells to $96.5 \pm 1.7$%, the antibody-conjugated formulation exhibited a higher impact. (HSA-NP)-IgG obtained the lowest cell viability with $77.7 \pm 9.6$%. Nonetheless, the high standard deviations of the Jurkat cell viabilities for the highest NP concentration had to be respected resulting in no significance and therefore only a slight viability reduction. As observed for hCMEC/D3 cells, pure DTPA led to the highest decrease of cell viability to $73.0 \pm 6.6$%. For primary macrophages and HUVECs the cell viability was not influenced by the NP, maintaining values around 100% (**Figure 3C**).

**Example 4b** Cellular interaction

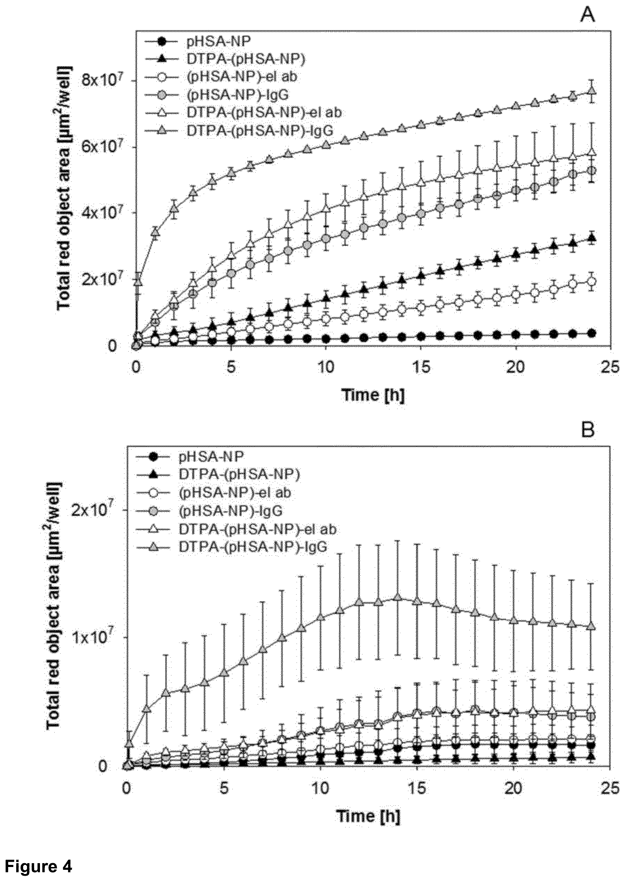

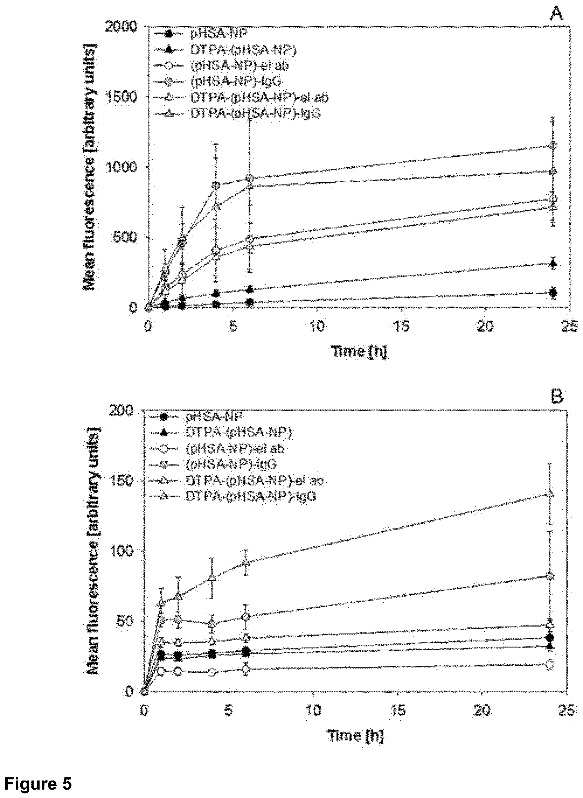

Precondition for the NP to reach the calcified elastic fibers is little to no interaction with endothelial cells or components of the blood. Live-cell imaging experiments showed a higher NP interaction with hCMEC/D3 cells (**Figure 4A**) compared to Jurkat cells (**Figure 4B**). However, the ranking of the interaction of the different NP formulations was comparable for both cell lines. DTPA-(pHSA-NP)-IgG showed the highest interaction followed by the el ab-bound formulations, while NP without conjugated antibody interacted less. Additionally, FACS analysis was performed to support the obtained live cell imaging data. Shear forces occurring during FACS analysis washed off only loosely attached NP from the cells leading to results more comparable to those under the influence of blood stream. The results obtained via IncuCyte® were confirmed. hCMEC/D3 cells (**Figure 5A**) exhibited about ten times as much interaction as Jurkat cells (**Figure 5B**). Again, IgG conjugation led to the highest interaction followed by el ab-functionalized NP. NP missing a conjugated antibody interacted the least. For hCMEC/D3 cells, (pHSA-NP)-IgG and DTPA-(pHSA-NP)-IgG were significantly more associated to cells than HSA-NP and DTPA-(pHSA-NP), respectively, after 24 h (p ≤ 0.05). IgG-bound NP had interacted significantly more with Jurkat cells after 24 h as well (DTPA-(pHSA-NP)-IgG to DTPA-(pHSA-NP): p ≤ 0.001; (pHSA-NP)-IgG to pHSA-NP: p ≤ 0.01). For both cell lines conjugation of the anti-elastin antibody to DTPA-(pHSA-NP) resulted in significantly less cellular interaction than DTPA-(pHSA-NP)-IgG (p ≤ 0.001). Fluorescence microscopy visually confirmed the quantification data (**Figures 6 and 7**). Cells without nanoparticle incubation served as control and exhibited no red fluorescence (data not shown). pHSA-NP (**Figure 6A**) showed no interaction with hCMEC/D3 cells and only occasional red fluorescent NP could be detected. DTPA-(pHSA-NP) (**Figure 6D**) seemed to cover the cells in a haze rather than showing cell interaction. Antibody-conjugated nanoparticles (pHSA-NP)-ab (**Figure 6B and C**), DTPA-(pHSA-NP)-ab (**Figures 6E and F**) showed more cellular interaction although it appeared to be more membrane associated rather than an uptake of the NP. For Jurkat cells (**Figure 7**) very little NP could be detected. Only IgG-conjugated nanoparticles (**Figures 7C and F**) were localized at the cells. Briefly summarized, all experiments to evaluate the cellular interaction revealed only little cell association.

**Example 5** Establishment and characterization of an *in vivo* mouse model displaying calcified subretinal pigment epithelial deposits resembling calcified drusen and drusen like calcifications

In 2022, Lengyel *et al.* reported Bruch's membrane (BM) calcification in ENPP1 deficient mice. ENPP1 transgenic mice were generated by breeding mice with loxP sites flanking ENPP1 exon 9 with mice expressing Cre recombinase behind a CAG synthetic germline promoter. BM calcification was observed in mice carrying the homozygous exon 9 deletion at 6 months of age (Lengyel *et al.,* 2022). To generate a mouse model with robust subretinal pigment epithelial deposits similar to calcified drusen and drusen like calcifications the inventors used ttw/ttw mice. These mice are carrying a homozygous naturally occurring nonsense mutation in the *Enpp1* gene (Okawa *et al.,* 1998) and were subjected to a diet with a high phosphorus and low magnesium content starting at 5 weeks of age to accelerate the calcification process. Ttw/ttw mice on this acceleration diet developed ocular calcifications as early as at the age of 9 weeks. Ocular calcifications were even more profound at 12 weeks of age (**Figure 8**). Calcifications presented as drusenoid solid deposits at the interface between the choroid and the RPE (**Figures 9 and 10**).

X-ray microscopy of ttw/ttw and WT eyes are shown in **Figure 11.** Reconstruction of the whole eye reveals a distribution of small mineral deposits in the ttw/ttw eye that extend both around the ciliary body of the eye and along major arcs extending towards the macula (**Figure 11A**). These calcified particles appear similar to hard drusen in both size (Pedersen *et al.,* 2018) and morphology (Suzuki *et al.,* 2015), which can be an early hallmark of macular degeneration (Sarks, 1980). Higher-resolution imaging of the retina in zones without the drusenoid bodies show the clear stratification of the retina (**Figure 11B**), allowing for an approximate 3D localization of the calcified drusenoid deposits. Zones containing the calcified drusenoid deposits are primarily located beneath the choroid, perturbing both Bruch's membrane and the retinal pigment epithelium (**Figure 11C**). On the scan of the full ttw/ttw eye, the inventors were able to segment 65 unique drusenoid deposits for an examination of their size (**Figure 11D**). The median drusenoid deposit in the eye of the 12-week-old ttw/ttw mouse was measured to have a volume of 4200 $\mu m^3$ and an average Feret diameter of 25 $\mu m$. Across this whole ttw/ttw eye, the accumulation of drusenoid deposits accounted for roughly 390,000 $\mu m^3$ in calcified tissue volume. Examination of a comparable WT mouse is shown in **Figure 11E,** where no drusenoid features are present anywhere in the eye.

Scanning electron microscopy of a drusenoid body/calcified deposit in the posterior segment of a TTW eye is shown in **Figure 12A-G** **Figure 12B** shows a small region containing the calcified deposits, where the retinal pigment epithelium appears to be partially atrophied directly surrounding the drusen compared to an unmineralized region of the same eye (**Figure 12C**) - seen similarly in histology by Tan *et al.* (Tan *et al.*, 2018). Energy dispersive X-ray spectroscopy maps of the drusen-containing region (**Figure 12D-E**) show an abundance of calcium and phosphorus within the deposit, alongside some slightly elevated phosphorus in the outer nuclear layer of the retina. There does not appear to be any microscale accumulation of calcium in a drusen-deficient region of the ttw/ttw eye, although the phosphorus content of the outer nuclear layer remains visible (**Figure 12F-G**). Pilgrim *et al.* have performed EDX of human drusen to find elevated calcium and phosphorus, although our findings lack the distinct spherical particulate texture present in their drusen (Pilgrim *et al.,* 2021). X-ray diffraction on a calcified deposit in the epoxy embedded ttw/ttw eye is shown in **Figure 12H.** The presence of arcs in place of strong diffraction spots indicates a polycrystalline material composing the deposit. The integrated diffraction spectra show some peaks [(002), (022), (130), (052), and (342)/(160)] that likely correspond to a hydroxyapatite or carbonate-substituted hydroxyapatite phase alongside unidentifiable strong peaks at 36.24° (d = 2.48 Å) and 42.10° (d = 2.14 Å) - potentially contribution from a whitlockite or brushite phase. The peaks at [(002), (022), (130), (052), and (342)/(160)] are the so-called Miller indices that refer to specific atomic crystalline planes found in crystalline materials, and they are characteristic for specific mineral phases. The D-spacings, (d = 2.48 Å) and (d = 2.14 Å) mentioned above, are the actual spacing distances between adjacent parallel crystal lattice planes of atoms whose distances are characteristic for a mineral phase.

Light microscopy image of a drusenoid calcified deposit near the ora serrata shows that the mineral lies between the choriocapillaris and the RPE (**Figure 13**). TEM images reveal that the deposit has a globular shape, lies outside of a vessel, separating the RPE from Bruch's membrane (**Figure 14**). Light microscopy image of a more flat drusenoid calcified deposit along the posterior-oriented arc of a ttw/ttw eye again shows that the mineral lies in the subretinal pigment epithelial space (**Figure 15**). TEM figures demonstrate that the Bruch's membrane is seemingly enveloped by the calcified deposit (**Figure 16**). **Figures 13-16** clearly demonstrate the localization of the deposits in the subretinal pigment epithelial space, while the EDX results document the polycrystalline content of the deposits (**Figure 12**). Microanatomic localization, and content of the identified deposits in ttw/ttw mice on a high phosphate diet therefore correspond very well to the localization and content of calcified drusen in the context of AMD.

**Example 8** Accumulation of magnetite-HSA-NP-el ab at the calcified Bruch's membrane of ttw/ttw mice

To show if nanoparticles comprising HSA and the elastin antibody could reach the calcified Bruch's membrane of ttw/ttw mice on acceleration diet (see above), the inventors prepared magnetite nanoparticles comprising HSA and the elastin antibody to enable electron microscopic imaging. The magnetite-HSA-NP-el ab were injected via the tail vein into ttw/ttw mice on acceleration diet. 4 hours or 48 hours after the intravenous injection the mice were sacrificed and the eye tissue prepared as described above. Using an electron microscope, the magnetite-HSA-NP-el ab could be seen as black dots at the Bruch's membrane of ttw/ttw mice (**Figure 17**). Thus, magnetite-HSA-NP-el ab are able to accumulate at the calcified Bruch's membrane of ttw/ttw mice.

**Example 9** Dissolution of BM calcifications and drusen like calcifications *in vivo*

The nanoparticles used in Example 9 were prepared according to point 1.2.5 of the Materials and Methods section. To investigate the potential of DTPA bound HSA based nanoparticles coupled to anti-elastin antibodies to resolve existing calcifications in subretinal pigment epithelial deposits *in vivo,* the inventors started to inject the DTPA bound HSA-based nanoparticles containing an anti-elastin antibody systemically into the lateral tail vein of ttw/ttw mice on acceleration diet at 10 weeks of age at a dose of 20 mg/kg. At this time point, ectopic calcifications were already reliably present in the eyes of this animal model (Figure 8). Mice were injected intravenously (i.v.) twice a week with the same dose into the lateral tail vein (4 injections in total) until they were sacrificed at the age of 12 weeks. Systemic administration of the NP formulation led to a significant dissolution of ectopic calcifications of the heart (p ≤ 0.05), the sheaths of the vibrissae (p ≤ 0.0005), the spleen (p ≤ 0.05) and the femoral arteries (p ≤ 0.005) in ttw/ttw mice subjected to an acceleration diet (**Figure 18**). Systemic administration of the NP formulation also led to a dissolution of ocular calcification in subretinal pigment epithelial deposits in this model, which was highly significant (p ≤ 0.0005) (**Figure 19**). The degree of ocular calcification after the administration of the NP formulation in the ttw/ttw mouse model did not differ from the degree of ocular calcification observed in wild-type mice of the same age (**Figure 19**).

**REFERENCES**

Arya S, Emri E, Synowsky SA, Shirran SL, Barzegar-Befroei N, Peto T, Botting CH, Lengyel I, Stewart AJ. Quantitative analysis of hydroxyapatite-binding plasma proteins in genotyped individuals with late-stage age-related macular degeneration. Exp Eye Res. 2018 Jul;172:21-29. Epub 2018 Mar 24.

Bentin JM, Heegaard S, Jørgensen NR, Grahnemo L, Hamann S. Optic disc drusen: Dystrophic calcification, a potential target for treatment. Eye (Lond). 2024 Aug;38(12):2359-2364. Epub 2024 May 22.

Bergen AA, Arya S, Koster C, Pilgrim MG, Wiatrek-Moumoulidis D, van der Spek PJ, Hauck SM, Boon CJF, Emri E, Stewart AJ, Lengyel I. On the origin of proteins in human drusen: The meet, greet and stick hypothesis. Prog Retin Eye Res. 2019 May;70:55-84. Epub 2018 Dec 17.

Bertrand J, Nitschke Y, Fuerst M, Hermann S, Schäfers M, Sherwood J, Nalesso G, Ruether W, Rutsch F, Dell'Accio F, Pap T. Decreased levels of nucleotide pyrophosphatase phosphodiesterase 1 are associated with cartilage calcification in osteoarthritis and trigger osteoarthritic changes in mice. Ann Rheum Dis. 2012 Jul;71 (7):1249-53.

Biesemeier A, Yoeruek E, Eibl O, Schraermeyer U. Iron accumulation in Bruch's membrane and melanosomes of donor eyes with age-related macular degeneration. Exp Eye Res. 2015 Aug;137:39-49.

Booij JC, Baas DC, Beisekeeva J, Gorgels TG, Bergen AA. The dynamic nature of Bruch's membrane. Prog Retin Eye Res. 2010 Jan;29(1):1-18.

Boskey AI, Posner AS. Formation of hydroxyapatite at low supersaturation. J Phys Chem 1976;80(1):40-45

Bäck M, Aranyi T, Cancela ML, Carracedo M, Conceição N, Leftheriotis G, Macrae V, Martin L, Nitschke Y, Pasch A, Quaglino D, Rutsch F, Shanahan C, Sorribas V, Szeri F, Valdivielso P, Vanakker O, Kempf H. Endogenous Calcification Inhibitors in the Prevention of Vascular Calcification: A Consensus Statement From the COST Action EuroSoftCalcNet. Front Cardiovasc Med. 2019 Jan 18;5:196.

Gorgels TG, Teeling P, Meeldijk JD, Nillesen ST, van der Wal AC, van Kuppevelt TH, Bergen AA. Abcc6 deficiency in the mouse leads to calcification of collagen fibers in Bruch's membrane. Exp Eye Res. 2012 Nov;104:59-64.

Hamann S, Malmqvist L, Costello F. Optic disc drusen: understanding an old problem from a new perspective. Acta Ophthalmol. 2018 Nov;96(7):673-684. Epub 2018 Apr 16.

Hnatowich DJ, Layne WW, Childs RL, Lanteigne D, Davis MA, Griffin TW, Doherty PW. Radioactive labeling of antibody: a simple and efficient method. Science. 1983 May 6;220(4597):613-5.

Hosoda Y, Yoshimura Y, Higaki S. A new breed of mouse showing multiple osteochondral lesions--twy mouse. Ryumachi. 1981;21 Suppl:157-64.

Katz BJ, Pomeranz HD. Visual field defects and retinal nerve fiber layer defects in eyes with buried optic nerve drusen. Am J Ophthalmol. 2006 Feb;141(2):248-253.

Kim H, Robinson SB, Csaky KG. Investigating the movement of intravitreal human serum albumin nanoparticles in the vitreous and retina. Pharm Res. 2009 Feb;26(2):329-37.

Keuth J, Nitschke Y, Mulac D, Riehemann K, Rutsch F, Langer K. Reversion of arterial calcification by elastin-targeted DTPA-HSA nanoparticles. Eur J Pharm Biopharm. 2020 May;150:108-119.

LaRusso J, Li Q, Jiang Q, Uitto J. Elevated dietary magnesium prevents connective tissue mineralization in a mouse model of pseudoxanthoma elasticum (Abcc6(-/-)). J Invest Dermatol. 2009 Jun;129(6):1388-94.

Lengyel I, Brown CN, Augustine J, Friedel T, Cunningham F, MacRae V, Kortvely E, Pilgrim M. Ectopic calcification in the Bruch's membrane and functional changes in the retina are associated with exon 9 deletion in ENPP1 in a transgenic mice model. Invest. Ophthalmol. Vis. Sci. 2022;63(7):464 - A0001 (abstract)

Liu J, Laiginhas R, Shen M, Shi Y, Li J, Trivizki O, Waheed NK, Gregori G, Rosenfeld PJ. Multimodal Imaging and En Face OCT Detection of Calcified Drusen in Eyes with Age-Related Macular Degeneration. Ophthalmol Sci. 2022 Jun;2(2):100162. Epub 2022 Apr 20.

Marty JJ, Oppenheim RC, Speiser P. Nanoparticles--a new colloidal drug delivery system. Pharm Acta Helv. 1978;53(1):17-23.

Mesken J, Iltzsche A, Mulac D, Langer K. Modifying plasmid-loaded HSA-nanoparticles with cell penetrating peptides - Cellular uptake and enhanced gene delivery. Int J Pharm. 2017 Apr 30;522(1-2):198-209

Michaelis M, Langer K, Arnold S, Doerr HW, Kreuter J, Cinatl J Jr. Pharmacological activity of DTPA linked to protein-based drug carrier systems. Biochem Biophys Res Commun. 2004 Oct 29;323(4):1236-40.

Najjar DM, Cohen EJ, Rapuano CJ, Laibson PR. EDTA chelation for calcific band keratopathy: results and long-term follow-up. Am J Ophthalmol. 2004 Jun;137(6):1056-64.

Okawa A, Nakamura I, Goto S, Moriya H, Nakamura Y, Ikegawa S. Mutation in Npps in a mouse model of ossification of the posterior longitudinal ligament of the spine. Nat Genet. 1998 Jul;19(3):271-3

Ouyang Y, Heussen FM, Hariri A, Keane PA, Sadda SR. Optical coherence tomography-based observation of the natural history of drusenoid lesion in eyes with dry age-related macular degeneration. Ophthalmology. 2013 Dec;120(12):2656-2665. Epub 2013 Jul 4.

Palmer E, Gale J, Crowston JG, Wells, AP. Optic Nerve Head Drusen: An Update. Neuroopthalmology 2018 Apr;42(6): 367-384

Paul H, Reginato AJ, Schumacher HR. Alizarin red S staining as a screening test to detect calcium compounds in synovial fluid. Arthritis Rheum. 1983 Feb;26(2):191-200.

Pedersen HR, Gilson SJ, Dubra A, Munch IC, Larsen M, Baraas RC. Multimodal imaging of small hard retinal drusen in young healthy adults. Br J Ophthalmol. 2018 Jan;102(1):146-152. Epub 2017 Oct 19.

Pilgrim MG, Marouf S, Fearn S, Csincsik L, Kortvely E, Knowles JC, Malek G, Thompson RB, Lengyel I. Characterization of Calcium Phosphate Spherical Particles in the Subretinal Pigment Epithelium-Basal Lamina Space in Aged Human Eyes. Ophthalmol Sci. 2021 Aug 19;1(3):100053.

Raming K, Künzel SH, Pfau M, Hendig D, Holz FG, Pfau K. Optic Disc Drusen in Pseudoxanthoma Elasticum Are Associated with the Extent of Bruch's Membrane Calcification. J Clin Med. 2024 Jun 10;13(12):3395.

Roll P, Schmut O, Wutz W, Hofmann H. Die Wirkung von Äthylendiamintetraessigsäure auf die Retina. Elektronenoptische Studie [The action of ethylenediaminetetraacetate on the retina. Electronmicroscopic study (author's transl)]. Albrecht Von Graefes Arch Klin Exp Ophthalmol. 1977 May 23;202(3):205-12.

Saito E, Suarez-Gonzalez D, Rao RR, Stegemann JP, Murphy WL, Hollister SJ. Use of micro-computed tomography to nondestructively characterize biomineral coatings on solid freeform fabricated poly (L-lactic acid) and poly (($\varepsilon$-caprolactone) scaffolds in vitro and in vivo. Tissue Eng Part C Methods. 2013 Jul;19(7):507-17.

Sarkar BC, Chauhan UP. A new method for determining micro quantities of calcium in biological materials. Anal Biochem. 1967 Jul;20(1):155-66.

Sarks SH. Council Lecture. Drusen and their relationship to senile macular degeneration. Aust J Ophthalmol. 1980 May;8(2):117-30.

Singh SR, Grossniklaus HE, Kang SJ, Edelhauser HF, Ambati BK, Kompella UB. Intravenous transferrin, RGD peptide and dual-targeted nanoparticles enhance anti-VEGF intraceptor gene delivery to laser-induced CNV. Gene Ther. 2009 May;16(5):645-59

Spraul CW, Lang GE, Grossniklaus HE, Lang GK. Histologic and morphometric analysis of the choroid, Bruch's membrane, and retinal pigment epithelium in postmortem eyes with age-related macular degeneration and histologic examination of surgically excised choroidal neovascular membranes. Surv Ophthalmol. 1999 Oct;44 Suppl 1:S10-32

Suzuki M, Curcio CA, Mullins RF, Spaide RF. REFRACTILE DRUSEN: Clinical Imaging and Candidate Histology. Retina. 2015 May;35(5):859-65.

Szmacinski H, Hegde K, Zeng HH, Eslami K, Puche AC, Lengyel I, Thompson RB. Imaging hydroxyapatite in sub-retinal pigment epithelial deposits by fluorescence lifetime imaging microscopy with tetracycline staining. J Biomed Opt. 2020 Apr;25(4):1-11

**EP 4 635 518 A1**

Tan ACS, Pilgrim MG, Fearn S, Bertazzo S, Tsolaki E, Morrell AP, Li M, Messinger JD, Dolz-Marco R, Lei J, Nittala MG, Sadda SR, Lengyel I, Freund KB, Curcio CA. Calcified nodules in retinal drusen are associated with disease progression in age-related macular degeneration. Sci Transl Med. 2018 Nov 7;10(466):eaat4544.

Thompson RB, Reffatto V, Bundy JG, Kortvely E, Flinn JM, Lanzirotti A, Jones EA, McPhail DS, Fearn S, Boldt K, Ueffing M, Ratu SG, Pauleikhoff L, Bird AC, Lengyel I. Identification of hydroxyapatite spherules provides new insight into subretinal pigment epithelial deposit formation in the aging eye. Proc Natl Acad Sci U S A. 2015 Feb 3;112(5):1565-70.

van der Schaft TL, Mooy CM, de Bruijn WC, Oron FG, Mulder PG, de Jong PT. Histologic features of the early stages of age-related macular degeneration. A statistical analysis. Ophthalmology. 1992 Feb;99(2):278-86

Weber C, Kreuter J, Langer K. Desolvation process and surface characteristics of HSA-nanoparticles. Int J Pharm. 2000 Mar 10;196(2):197-200

Weber C, Reiss S, Langer K. Preparation of surface modified protein nanoparticles by introduction of sulfhydryl groups. Int J Pharm. 2000 Dec 15;211(1-2):67-78 (=Weber et al., 2000a)

Yamaguchi A, Rajput AR, Ohzeki R, Kambara T. Determination of Ethylenediaminetetraacetic Acid and Nitrilotriacetic Acid as Their Iron(III)-Complexes by Reversed Phase High Performance Liquid Chromatography. Bull. Chem. Soc. Jpn. 1983;56(9):2621-2623

**Claims**

1. A nanoparticle comprising a scaffold comprising a biodegradable material, an antibody, which is able to bind to a component of a Bruch's membrane, a component of a subretinal pigment epithelial deposit, or a component of an optic nerve head, and an anti-calcifying agent for use in a prophylaxis or in a treatment of age-related macular degeneration and/or optic nerve head drusen of an eye.

2. The nanoparticle for use of claim 1, wherein the prophylaxis or the treatment of age-related macular degeneration and/or optic nerve head drusen is accomplished by the prophylaxis or the treatment of a calcified subretinal pigment epithelium deposit and/or a calcified drusen of the eye.

3. The nanoparticle for use of any one of the preceding claims, wherein the biodegradable material is human serum albumin.

4. The nanoparticle for use of any one of the preceding claims, wherein the antibody, which is able to bind to the component of the Bruch's membrane, to the component of the subretinal pigment epithelial deposit, or to the component of the optic nerve head, is an antibody targeted to the component of the Bruch's membrane or targeted to a shell protein of hydroxyapatite spherules in the subretinal pigment epithelial deposit, or targeted to the component of the optic nerve head, preferably the antibody is an anti-elastin antibody, an anti-fibronectin antibody, an anti-complement factor H antibody, an anti-beta-amyloid antibody or an anti-vitronectin antibody, more preferably the antibody, which is able to bind to the component of the Bruch's membrane, the component of the subretinal pigment epithelial deposit, or to the component of the optic nerve head, is the anti-elastin antibody, and wherein the antibody, which is able to bind to the component of the Bruch's membrane, the component of the subretinal pigment epithelial deposit, or the component of the optic nerve head, is preferably covalently linked to the nanoparticle.

5. The nanoparticle for use of any one of the preceding claims, wherein the anti-calcifying agent is a chelator or an inorganic pyrophosphate (PPi), or a sodium thiosulfate, preferably the chelator is diethylenetriaminepentaacetic acid (DTPA) or ethylenediaminetetraacetic acid (EDTA).

6. The nanoparticle for use of any one of the preceding claims, wherein the nanoparticle has a negative surface charge.

7. The nanoparticle for use of any one of the preceding claims, wherein the nanoparticle comprises the scaffold comprising human serum albumin, the anti-elastin antibody preferably covalently linked to the nanoparticle, and DTPA preferably covalently linked to the nanoparticle.

8. A pharmaceutical composition comprising the nanoparticle of any one of the preceding claims and one or more pharmaceutical acceptable excipients.

9. The pharmaceutical composition of claim 8, for use in a prophylaxis or in a treatment of age-related macular degeneration and/or optic nerve head drusen of an eye.

10. The pharmaceutical composition for use of claim 9, wherein the prophylaxis or the treatment of age-related macular degeneration and/or optic nerve head drusen is accomplished by the prophylaxis or the treatment of a calcified subretinal pigment epithelium deposit and/or a calcified drusen of the eye.

11. The nanoparticle for use of any one of claims 1 to 7, the pharmaceutical composition of claim 8, or the pharmaceutical composition for use of any one of claims 9 or 10, wherein the nanoparticle or the pharmaceutical composition is administered intravenously, intravitreally, subconjunctivally, subtenonly, retrobulbarly, posteriorly, juxtasclerally, suprachoroideally, subretinally, or topically as eye drops.

**Figure 1**

**Figure 2**

Figure 3

**Figure 4**

**Figure 5**

**Figure 6**

**Figure 7**

**Figure 8**

**Figure 9**

DAPI    transmission light    osteosense    merge

**Figure 10**

**Figure 11**

Figure 12

Figure 13

Figure 14

**Figure 15**

**Figure 16**

**Figure 17**

**Figure 18**

Figure 19

Europäisches
Patentamt

European
Patent Office

Office européen
des brevets

**EUROPEAN SEARCH REPORT**

Application Number

EP 25 15 8185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KEUTH JACQUELINE ET AL: "Reversion of arterial calcification by elastin-targeted DTPA-HSA nanoparticles", EUROPEAN JOURNAL OF PHARMACEUTICS AND BIOPHARMACEUTICS, ELSEVIER SCIENCE PUBLISHERS B.V., AMSTERDAM, NL, vol. 150, 6 March 2020 (2020-03-06), pages 108-119, XP086129927, ISSN: 0939-6411, DOI: 10.1016/J.EJPB.2020.03.007 [retrieved on 2020-03-06] * paragraph [0002] * * paragraph [0005] * * figures; examples * | 1-11 | INV. A61K47/69 A61K9/00 A61K47/68 A61P27/02 |
| Y | HYUNCHEOL KIM ET AL: "Investigating the Movement of Intravitreal Human Serum Albumin Nanoparticles in the Vitreous and Retina", PHARMACEUTICAL RESEARCH, KLUWER ACADEMIC PUBLISHERS-PLENUM PUBLISHERS, NL, vol. 26, no. 2, 29 October 2008 (2008-10-29), pages 329-337, XP019686087, ISSN: 1573-904X * page 332, column 2 - page 336, column 1 * * abstract * | 1-11 | |

TECHNICAL FIELDS SEARCHED (IPC)

A61K

-/--

The present search report has been drawn up for all claims

| Place of search | Date of completion of the search | Examiner |
|---|---|---|
| The Hague | 29 August 2025 | Ceyte, Mathilde |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 25 15 8185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Richard B Thompson ET AL: "Identification of hydroxyapatite spherules provides new insight into subretinal pigment epithelial deposit formation in the aging eye", Proc Natl Acad Sci Proc Natl Acad Sci, 3 February 2015 (2015-02-03), pages 1565-1570, XP055252778, DOI: 10.1073/pnas.1413347112 Retrieved from the Internet: URL:http://www.pnas.org/content/112/5/1565.full.pdf [retrieved on 2016-02-24] * the whole document * | 1-11 | |

TECHNICAL FIELDS
SEARCHED       (IPC)

The present search report has been drawn up for all claims

| Place of search | Date of completion of the search | Examiner |
|---|---|---|
| The Hague | 29 August 2025 | Ceyte, Mathilde |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 24171280

**Non-patent literature cited in the description**

- **STEWART AJ**. Quantitative analysis of hydroxyapatite-binding plasma proteins in genotyped individuals with late-stage age-related macular degeneration. *Exp Eye Res.*, 24 March 2018, vol. 172, 21-29
- **BENTIN JM** ; **HEEGAARD S** ; **JØRGENSEN NR** ; **GRAHNEMO L** ; **HAMANN S**. Optic disc drusen: Dystrophic calcification, a potential target for treatment. *Eye (Lond)*, 22 May 2024, vol. 38 (12), 2359-2364
- **BERGEN AA** ; **ARYA S** ; **KOSTER C** ; **PILGRIM MG** ; **WIATREK-MOUMOULIDIS D** ; **VAN DER SPEK PJ** ; **HAUCK SM** ; **BOON CJF** ; **EMRI E** ; **STEWART AJ**. On the origin of proteins in human drusen: The meet, greet and stick hypothesis. *Prog Retin Eye Res.*, 17 December 2018, vol. 70, 55-84
- **BERTRAND J** ; **NITSCHKE Y** ; **FUERST M** ; **HERMANN S** ; **SCHÄFERS M** ; **SHERWOOD J** ; **NALESSO G** ; **RUETHER W** ; **RUTSCH F** ; **DELL'ACCIO F**. Decreased levels of nucleotide pyrophosphatase phosphodiesterase 1 are associated with cartilage calcification in osteoarthritis and trigger osteoarthritic changes in mice. *Ann Rheum Dis*, July 2012, vol. 71 (7), 1249-53
- **BIESEMEIER A** ; **YOERUEK E** ; **EIBL O** ; **SCHRAERMEYER U**. Iron accumulation in Bruch's membrane and melanosomes of donor eyes with age-related macular degeneration. *Exp Eye Res*, August 2015, vol. 137, 39-49
- **BOOIJ JC** ; **BAAS DC** ; **BEISEKEEVA J** ; **GORGELS TG** ; **BERGEN AA**. The dynamic nature of Bruch's membrane. *Prog Retin Eye Res*, January 2010, vol. 29 (1), 1-18
- **BOSKEY AL** ; **POSNER AS**. Formation of hydroxyapatite at low supersaturation. *J Phys Chem*, 1976, vol. 80 (1), 40-45
- **BÄCK M** ; **ARANYI T** ; **CANCELA ML** ; **CARRACEDO M** ; **CONCEIÇÃO N** ; **LEFTHERIOTIS G** ; **MACRAE V** ; **MARTIN L** ; **NITSCHKE Y** ; **PASCH A**. Endogenous Calcification Inhibitors in the Prevention of Vascular Calcification: A Consensus Statement From the COST Action EuroSoftCalcNet. *Front Cardiovasc Med*, 18 January 2019, vol. 5, 196
- **GORGELS TG** ; **TEELING P** ; **MEELDIJK JD** ; **NILLESEN ST** ; **VAN DER WAL AC** ; **VAN KUPPEVELT TH** ; **BERGEN AA**. Abcc6 deficiency in the mouse leads to calcification of collagen fibers in Bruch's membrane. *Exp Eye Res*, November 2012, vol. 104, 59-64
- **HAMANN S** ; **MALMQVIST L** ; **COSTELLO F**. Optic disc drusen: understanding an old problem from a new perspective. *Acta Ophthalmol*, 16 April 2018, vol. 96 (7), 673-684
- **HNATOWICH DJ** ; **LAYNE WW** ; **CHILDS RL** ; **LANTEIGNE D** ; **DAVIS MA** ; **GRIFFIN TW** ; **OHERTY PW**. Radioactive labeling of antibody: a simple and efficient method. *Science*, 06 May 1983, vol. 220 (4597), 613-5
- **HOSODA Y** ; **YOSHIMURA Y** ; **HIGAKI S**. A new breed of mouse showing multiple osteochondral lesions--twy mouse. *Ryumachi*, 1981 (21), 157-64
- **KATZ BJ** ; **POMERANZ HD**. isual field defects and retinal nerve fiber layer defects in eyes with buried optic nerve drusen. *Am J Ophthalmol.*, February 2006, vol. 141 (2), 248-253
- **KIM H** ; **ROBINSON SB** ; **CSAKY KG**. Investigating the movement of intravitreal human serum albumin nanoparticles in the vitreous and retina. *Pharm Res.*, February 2009, vol. 26 (2), 329-37
- **KEUTH J** ; **NITSCHKE Y** ; **MULAC D** ; **RIEHEMANN K** ; **RUTSCH F** ; **LANGER K**. Reversion of arterial calcification by elastin-targeted DTPA-HSA nanoparticles. *Eur J Pharm Biopharm*, May 2020, vol. 150, 108-119
- **LARUSSO J** ; **LI Q** ; **JIANG Q** ; **UITTO J.** Elevated dietary magnesium prevents connective tissue mineralization in a mouse model of pseudoxanthoma elasticum (Abcc6). *J Invest Dermatol.*, June 2009, vol. 129 (6), 1388-94
- **LENGYEL I** ; **BROWN CN** ; **AUGUSTINE J** ; **FRIEDEL T** ; **CUNNINGHAM F** ; **MACRAE V** ; **KORTVELY E** ; **PILGRIM M**. Ectopic calcification in the Bruch's membrane and functional changes in the retina are associated with exon 9 deletion in ENPP1 in a transgenic mice model.. *Invest. Ophthalmol. Vis. Sci.*, 2022, vol. 63 (7), 464

- **LIU J** ; **LAIGINHAS R** ; **SHEN M** ; **SHI Y** ; **LI J** ; **TRIVIZKI O** ; **WAHEED NK** ; **GREGORI G** ; **ROSENFELD PJ**. Multimodal Imaging and En Face OCT Detection of Calcified Drusen in Eyes with Age-Related Macular Degeneration. *Ophthalmol Sci.*, 20 April 2022, vol. 2 (2), 100162

- **MARTY JJ** ; **OPPENHEIM RC** ; **SPEISER P**. Nanoparticles--a new colloidal drug delivery system. *Pharm Acta Helv.*, 1978, vol. 53 (1), 17-23

- **MESKEN J** ; **ILTZSCHE A** ; **MULAC D** ; **LANGER K**. Modifying plasmid-loaded HSA-nanoparticles with cell penetrating peptides - Cellular uptake and enhanced gene delivery. *Int J Pharm.*, 30 April 2017, vol. 522 (1-2), 198-209

- **MICHAELIS M** ; **LANGER K** ; **ARNOLD S** ; **DOERR HW** ; **KREUTER J** ; **CINATL J JR.** Pharmacological activity of DTPA linked to protein-based drug carrier systems. *Biochem Biophys Res Commun.*, 29 October 2004, vol. 323 (4), 1236-40

- **NAJJAR DM** ; **COHEN EJ** ; **RAPUANO CJ** ; **LAIBSON PR**. EDTA chelation for calcific band keratopathy: results and long-term follow-up. *Am J Ophthalmol.*, June 2004, vol. 137 (6), 1056-64

- **OKAWA A** ; **NAKAMURA I** ; **GOTO S** ; **MORIYA H** ; **NAKAMURA Y** ; **IKEGAWA S**. Mutation in Npps in a mouse model of ossification of the posterior longitudinal ligament of the spine. *Nat Genet.*, July 1998, vol. 19 (3), 271-3

- **OUYANG Y** ; **HEUSSEN FM** ; **HARIRI A** ; **KEANE PA** ; **SADDA SR**. Optical coherence tomography-based observation of the natural history of drusenoid lesion in eyes with dry age-related macular degeneration. *Ophthalmology*, 04 July 2013, vol. 120 (12), 2656-2665

- **PALMER E** ; **GALE J** ; **CROWSTON JG** ; **WELLS, AP**. Optic Nerve Head Drusen: An Update. *Neuroopthalmology*, April 2018, vol. 42 (6), 367-384

- **PAUL H** ; **REGINATO AJ** ; **SCHUMACHER HR**. Alizarin red S staining as a screening test to detect calcium compounds in synovial fluid. *Arthritis Rheum*, February 1983, vol. 26 (2), 191-200

- **PEDERSEN HR** ; **GILSON SJ** ; **DUBRA A** ; **MUNCH IC** ; **LARSEN M** ; **BARAAS RC**. Multimodal imaging of small hard retinal drusen in young healthy adults. *Br J Ophthalmol.*, 19 October 2017, vol. 102 (1), 146-152

- **PILGRIM MG** ; **MAROUF S** ; **FEARN S** ; **CSINCSIK L** ; **KORTVELY E** ; **KNOWLES JC** ; **MALEK G** ; **THOMPSON RB** ; **LENGYEL I**. Characterization of Calcium Phosphate Spherical Particles in the Subretinal Pigment Epithelium-Basal Lamina Space in Aged Human Eyes. *Ophthalmol Sci.*, 19 August 2021, vol. 1 (3), 100053

- **RAMING K** ; **KÜNZEL SH** ; **PFAU M** ; **HENDIG D** ; **HOLZ FG** ; **PFAU K**. Optic Disc Drusen in Pseudoxanthoma Elasticum Are Associated with the Extent of Bruch's Membrane Calcification. *J Clin Med.*, 10 June 2024, vol. 13 (12), 3395

- **ROLL P** ; **SCHMUT O** ; **WUTZ W** ; **HOFMANN H**. Die Wirkung von Äthylendiamintetraessigsäure auf die Retina. Elektronenoptische Studie [The action of ethylenediaminetetraacetate on the retina. Electronmicroscopic study (author's transl). *Albrecht Von Graefes Arch Klin Exp Ophthalmol.*, 23 May 1977, vol. 202 (3), 205-12

- **SAITO E** ; **SUAREZ-GONZALEZ D** ; **RAO RR** ; **STEGEMANN JP** ; **MURPHY WL** ; **HOLLISTER SJ**. Use of micro-computed tomography to nondestructively characterize biomineral coatings on solid freeform fabricated poly (L-lactic acid) and poly ((ε-caprolactone) scaffolds in vitro and in vivo. *Tissue Eng Part C Methods*, July 2013, vol. 19 (7), 507-17

- **SARKAR BC** ; **CHAUHAN UP**. A new method for determining micro quantities of calcium in biological materials. *Anal Biochem.*, July 1967, vol. 20 (1), 155-66

- **SARKS SH**. Drusen and their relationship to senile macular degeneration. *Aust J Ophthalmol.*, May 1980, vol. 8 (2), 117-30

- **SINGH SR** ; **GROSSNIKLAUS HE** ; **KANG SJ** ; **EDELHAUSER HF** ; **AMBATI BK** ; **KOMPELLA UB**. Intravenous transferrin, RGD peptide and dual-targeted nanoparticles enhance anti-VEGF intraceptor gene delivery to laser-induced CNV. *Gene Ther.*, May 2009, vol. 16 (5), 645-59

- **SPRAUL CW** ; **LANG GE** ; **GROSSNIKLAUS HE** ; **LANG GK**. Histologic and morphometric analysis of the choroid, Bruch's membrane, and retinal pigment epithelium in postmortem eyes with age-related macular degeneration and histologic examination of surgically excised choroidal neovascular membranes. *Surv Ophthalmol*, October 1999, vol. 44 (1), 10-32

- **SUZUKI M** ; **CURCIO CA** ; **MULLINS RF** ; **SPAIDE RF**. REFRACTILE DRUSEN: Clinical Imaging and Candidate Histology. *Retina*, May 2015, vol. 35 (5), 859-65

- **SZMACINSKI H** ; **HEGDE K** ; **ZENG HH** ; **ESLAMI K** ; **PUCHE AC** ; **LENGYEL I** ; **THOMPSON RB**. Imaging hydroxyapatite in sub-retinal pigment epithelial deposits by fluorescence lifetime imaging microscopy with tetracycline staining. *J Biomed Opt.*, April 2020, vol. 25 (4), 1-11

- **TAN ACS** ; **PILGRIM MG** ; **FEARN S** ; **BERTAZZO S** ; **TSOLAKI E** ; **MORRELL AP** ; **LI M** ; **MESSINGER JD** ; **DOLZ-MARCO R** ; **LEI J**. Calcified nodules in retinal drusen are associated with disease progression in age-related macular degeneration. *Sci Transl Med.*, 07 November 2018, vol. 10 (466), eaat4544

- **THOMPSON RB** ; **REFFATTO V** ; **BUNDY JG** ; **KORTVELY E** ; **FLINN JM** ; **LANZIROTTI A** ; **JONES EA** ; **MCPHAIL DS** ; **FEARN S** ; **BOLDT K**. Identification of hydroxyapatite spherules provides new insight into subretinal pigment epithelial deposit formation in the aging eye. *Proc Natl Acad Sci U S A.*, 03 February 2015, vol. 112 (5), 1565-70
- **VAN DER SCHAFT TL** ; **MOOY CM** ; **DE BRUIJN WC** ; **ORON FG** ; **MULDER PG** ; **DE JONG PT**. Histologic features of the early stages of age-related macular degeneration. A statistical analysis. *Ophthalmology*, February 1992, vol. 99 (2), 278-86

- **WEBER C** ; **KREUTER J** ; **LANGER K**. Desolvation process and surface characteristics of HSA-nanoparticles. *Int J Pharm.*, 10 March 2000, vol. 196 (2), 197-200
- **WEBER C** ; **REISS S** ; **LANGER K**. Preparation of surface modified protein nanoparticles by introduction of sulfhydryl groups. *Int J Pharm.*, 15 December 2000, vol. 211 (1-2), 67-78
- **YAMAGUCHI A** ; **RAJPUT AR** ; **OHZEKI R** ; **KAMBARA T**. Determination of Ethylenediaminetetraacetic Acid and Nitrilotriacetic Acid as Their Iron(III)-Complexes by Reversed Phase High Performance Liquid Chromatography. *Bull. Chem. Soc. Jpn.*, 1983, vol. 56 (9), 2621-2623